# EUROPEAN PATENT APPLICATION

(11) **EP 4 745 980 A1**
(43) Date of publication of application: **20.05.2026**
(21) Application number: 25215168.3
(22) Date of filing: 12.11.2025
(51) Int. Cl.: G16H 20/30, G16H 50/20, G16H 10/40, G16H 40/67

(54) **SYSTEM AND METHOD FOR SUPPORTING HEALTH MANAGEMENT OF AN ATHLETIC PLAYER**

(30) Priority: 15.11.2024 JP 2024200048
(71) Applicant: SYSMEX CORPORATION, Kobe-shi Hyogo 651-0073 (JP)
(72) Inventor: OTSUKA, Shunsuke, Tokyo, 195-0055 (JP); KAWASHIMA, Yasuyuki, Hyogo, 651-0073 (JP); ENDOH, Takashi, Hyogo, 651-0073 (JP); ADACHI, Akiko, Hyogo, 651-0073 (JP)
(74) Representative: Becker, Eberhard

(57) **Abstract**

Disclosed is a system (1) for supporting health management of an athletic player, comprising: a measuring device (40) configured to measure myoglobin contained in blood of the player; and a server (10) configured to record an exercise load of the player and a measured value of the myoglobin of the player, wherein the server is configured to provide the exercise load of the player and the measured value of the player in response to a request from a terminal (20, 30).

## Description

### CROSS REFERENCE TO RELATED APPLICATIONS

This application claims priority from prior Japanese Patent Application No. 2024-200048, filed on November 15, 2024, entitled "SYSTEM AND METHOD", the entire content of which is incorporated herein by reference.

### FIELD OF THE INVENTION

The present invention relates to a system and the like for supporting health management of an athletic player.

### BACKGROUND OF THE INVENTION

Health management of an athletic player is important for the player himself/herself and also for managers such as a coach or an athletic director. For example, JP2008-293338 discloses a technique for proposing necessary exercise based on a user's calorie information while considering the user's physical condition information.

### SUMMARY OF THE INVENTION

Conventional techniques, including the technique disclosed in JP2008-293338, are not sufficient in terms of supporting the health management of an athletic player.

The present invention has been made in view of the above-described problems, and one of its objects is to propose a novel method for supporting the health management of an athletic player.

According to a first aspect of the present invention, a system for supporting health management of an athletic player comprises: a measuring device for measuring myoglobin contained in blood of the player; and a server configured to record an exercise load of the player and a measured value of the myoglobin of the player, wherein the server is configured to provide the exercise load of the player and the measured value of the player in response to a request from a terminal.
According to a second aspect of the present invention, a method for supporting health management of an athletic player includes: measuring myoglobin contained in blood of the player; recording an exercise load of the player and a measured value of the myoglobin of the player; and providing the exercise load of the player and the measured value of the player in response to a request from a terminal.

According to the present invention, it is possible to output the exercise load and the measured value of myoglobin of an athletic player to a terminal, thereby supporting the health management of the athletic player.

### BRIEF DESCRIPTION OF THE DRAWINGS

FIG. 1 is a diagram showing an example of a system configuration in an embodiment.
FIG. 2 is a diagram showing an example of a functional configuration of a sample measuring device.
FIG. 3 is a diagram showing an example of a configuration of a container (cartridge).
FIG. 4 is a diagram showing an example of a functional configuration of a controller of a server.
FIG. 5 is a diagram showing an example of information stored in a storage of a server.
FIG. 6 is a diagram showing an example of a data configuration of team information registration data.
FIG. 7 is a diagram showing an example of a data configuration of a player information management database.
FIG. 8 is a diagram showing an example of a functional configuration of a controller of a player terminal.
FIG. 9 is a diagram showing an example of information stored in a storage of a player terminal.
FIG. 10 is a diagram showing an example of a display screen displayed on a display of a player terminal.
FIG. 11 is a diagram showing an example of a display screen displayed on a display of a player terminal.
FIG. 12 is a diagram showing an example of a display screen displayed on a display of a player terminal.
FIG. 13 is a diagram showing an example of a display screen displayed on a display of a player terminal.
FIG. 14 is a diagram showing an example of a display screen displayed on a display of a manager terminal.
FIG. 15 is a diagram showing an example of a display screen displayed on a display of a manager terminal.
FIG. 16 is a diagram showing an example of a display screen displayed on a display of a manager terminal.
FIG. 17 is a flowchart showing an example of a flow of a process executed by each device of the system.
FIG. 18 is a diagram showing a specific example of myoglobin measurement results and playing time obtained multiple times for a plurality of players.
FIG. 19 is a flowchart showing an example of a flow of an exercise load registration process.
FIG. 20 is an explanatory diagram of registration of a myoglobin measurement result.
FIG. 21 is an explanatory diagram of a measurement date and time of myoglobin.
FIG. 22 is an explanatory diagram of a method for determining a myoglobin score.
FIG. 23 is a flowchart showing an example of a flow of a recommended training menu determination process.
FIG. 24 is a flowchart showing an example of a flow of a recommended training menu determination process.
FIG. 25 is a diagram showing an example of a display screen displayed on a display of a manager terminal.
FIG. 26 is a flowchart showing an example of a flow of a process executed by each device of the system.
FIG. 27 is a flowchart showing an example of a flow of a process executed by each device of the system.
FIG. 28 is a diagram showing an example of a display screen displayed on a display of a manager terminal.
FIG. 29 is an explanatory diagram of a method for calculating a fatigue level score.
FIG. 30 is an explanatory diagram of a method for determining a recommended training menu.

### DETAILED DESCRIPTION

An embodiment for implementing a system and the like according to the present disclosure will be described with reference to the drawings.
Note that in the description of the drawings, the same elements are denoted by the same reference numerals, and redundant description may be omitted.
Also, the components described in this embodiment are merely examples, and not intended to limit the scope of the present invention to them.

The system 1 of the present embodiment may be an example of a system for supporting physical condition management of a human (health management system), for example.
The system 1 may be configured to include a plurality of devices, for example, and the plurality of devices may cooperate to perform some process.
Furthermore, the system 1 may be configured as a client-server system, for example, and may be a system including at least one terminal and at least one server.

The server may be a single device or a combination of a plurality of devices (server system). Furthermore, the function of the server may be provided in the form of PaaS, IaaS, or SaaS in cloud computing.

The system 1 may also be a system configured by a plurality of terminals. This system may be, for example, the following system:
- A system in which the function of the server is held by the terminal (distributed system). This can be realized, for example, using blockchain technology.
- A system in which terminals perform wireless communication with each other. This can be realized, for example, by performing communication in a P2P (peer-to-peer) method using short-range wireless communication technology such as Bluetooth (registered trademark).

### <System Configuration>

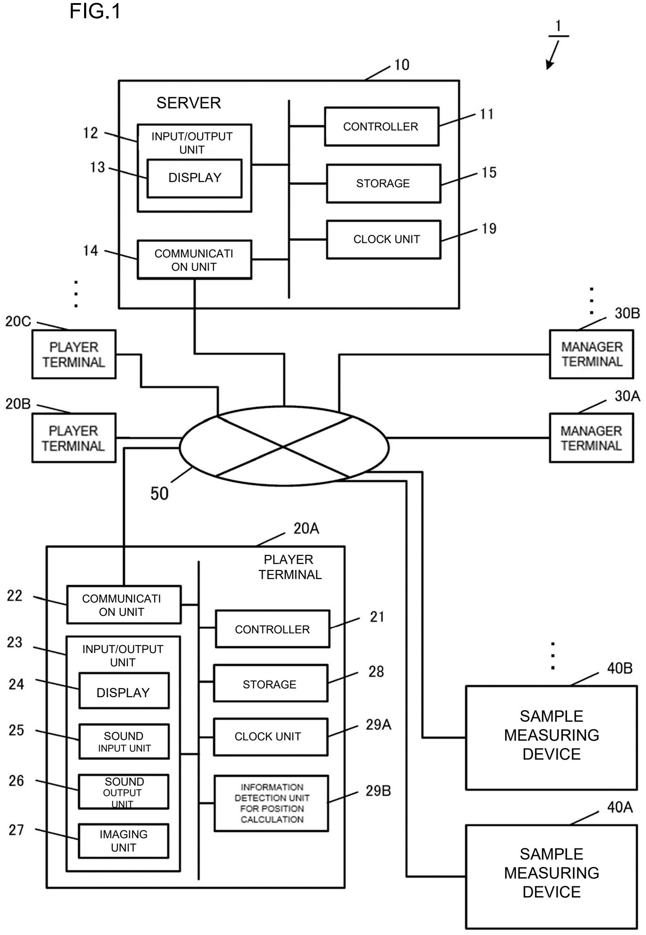

FIG. 1 is a diagram showing an example of the system configuration of the system 1 in the present embodiment.
In the system 1, for example, a server 10, one or more player terminals 20 (20A, 20B, 20C, ...), one or more manager terminals 30 (30A, 30B, 30C, ...), and one or more sample measuring devices 40 (40A, 40B, 40C, ...)) are connected via a network 50.

The server 10 is an information processing device possessed by a predetermined operator, for example, and has a function of providing various information to various devices connected via the network 50 and a function of acquiring information from various devices connected via the network 50. As mentioned above, the server 10 may be a single or plurality of server devices.

The player terminal 20 is an information processing device used by an athletic player (sports player. Hereinafter referred to as a "player"), for example, and may be a smartphone, tablet terminal, personal computer, smartwatch, or the like.
In the present embodiment, soccer is exemplified as a sport. However, it is not limited to this and may be any sport.

The manager terminal 30 is a terminal (information processing device) used by a director or coach who manages the player, for example, and similar to the player terminal 20, it may be a smartphone, tablet terminal, personal computer, smartwatch, or the like.

The sample measuring device 40 is a device for measuring blood protein of a subject (the player in the present embodiment), for example, and may be a device configured to be capable of measuring the blood myoglobin value of the subject by sealing the subject's blood and setting it in the device. The sample measuring device 40 in the present embodiment measures myoglobin by mixing a reagent including a labeled antibody with blood to label the myoglobin with the labeled antibody and optically detecting the labeled myoglobin.
The sample measuring device 40 in the present embodiment may be a relatively small device that is portable by a user, for example.
It is known that when a player performs high-intensity exercise and muscle damage occurs, myoglobin flows out into the blood and the myoglobin value becomes higher compared to normal times. Therefore, by measuring the blood myoglobin value of the player, the degree of muscle fatigue of that player can be estimated. In other words, the myoglobin value may be considered as various quantities (parameters) related to muscle fatigue.

The network 50 plays the role of connecting each device constituting the system 1 and providing a connection path so that data can be transmitted and received. The network 50 may be a wide area network such as the Internet, which is composed of a wired network or a wireless network, for example.

Note that at least a part of the process in the server 10 and/or various terminals and/or the sample measuring device 40 may be realized by cloud computing composed of one or more computers.

Furthermore, unless explicitly mentioned, the configuration for evaluation in the embodiment of the present disclosure is not essential, and a predetermined process may be operated when the evaluation condition is satisfied, or a predetermined process may be performed when the evaluation condition is not satisfied.

### [Hardware Configuration of Each Device]

FIG. 1 shows an example of the hardware configuration of the server 10.
The server 10 comprises, for example, a controller 11 including a CPU or the like, a storage 15 including RAM, ROM, and a semiconductor drive, a communication unit 14 having wired or wireless communication functions, an input/output unit 12 composed of a keyboard or the like, a display 13 such as a liquid crystal display, and a clock unit 19 configured to include a clock utilizing a crystal oscillator or an NITC (Network Identity and Time Zone) clock or the like. Each component of the hardware of the server 10 is interconnected, for example, via a bus.

FIG. 1 shows an example of the hardware configuration of the player terminal 20.
The player terminal 20 comprises, for example, a controller 21, a storage 28, a communication unit 22, an input/output unit 23, a clock unit 29A, and a position calculation information detection unit 29B including a sensor and a GNSS unit for calculating the position of the player terminal 20 using a satellite positioning system such as GNSS (Global Navigation Satellite System). Each component of the hardware of the player terminal 20 is interconnected, for example, via a bus.

For example, the input/output unit 23 may include a display 24 composed of a liquid crystal display or OELD or the like, a sound input unit 25 composed of a microphone or the like, a sound output unit 26 composed of a speaker or the like, an imaging unit 27 composed of a camera or the like, and so on.

Note that the manager terminal 30 may be the same, although the illustration is omitted.

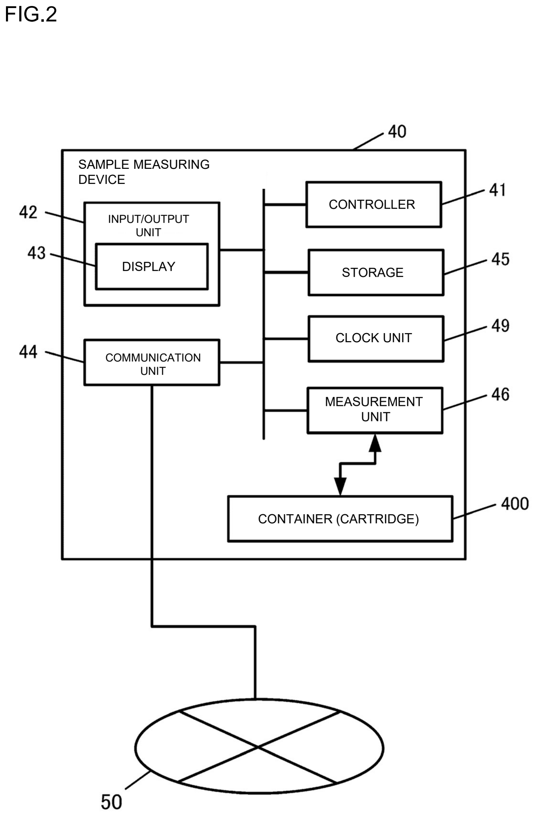

FIG. 2 shows an example of the hardware configuration of the sample measuring device 40.
The sample measuring device 40 comprises, for example, a controller 41, a storage 45, a communication unit 44, an input/output unit, a clock unit 49, a measurement unit 46, and a container 400 (cartridge), and so on.

For example, U.S. Patent No. 11327086 discloses a sample measuring device that measures a sample injected into a container using a container (for example, a disk-shaped cartridge) that has an accommodating portion for accommodating a reagent that reacts with the sample. By this sample measuring device, mixing, stirring, heating or cooling of the sample and the reagent, movement of a solid or liquid containing the sample, and various other operations are performed inside the container. U.S. Patent No. 11327086 is incorporated herein by reference.

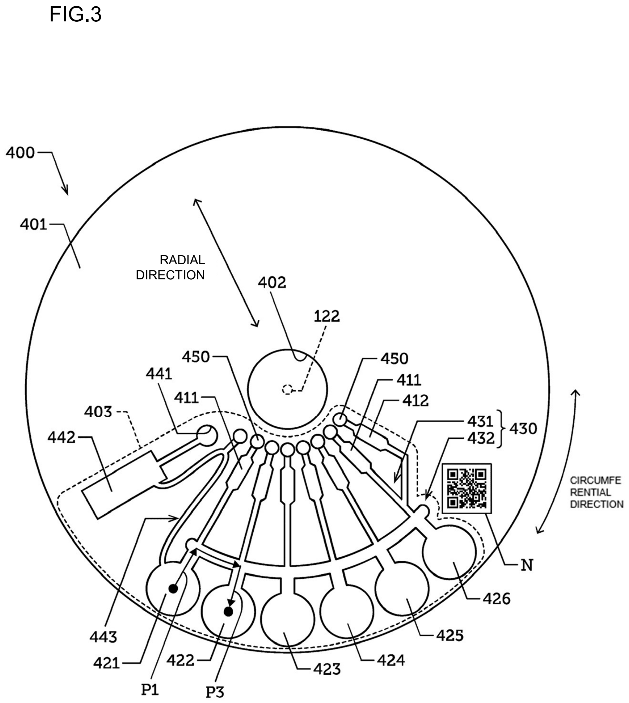

As shown in FIG. 3, the container 400 is a disk-type cartridge configured by a plate-shaped and disk-shaped substrate 401. Each part inside the container 400 is formed by laminating a recess formed in the substrate 401 and a film (not shown) that covers the entire surface including the recess of the substrate 401. The substrate 401 and the film laminated to the substrate 401 are composed of a member that has light permeability.

The substrate 401 is provided with a sample processing area 403 including a hole 402, six accommodating portions 411, one accommodating portion 412, six chambers 421 to 426, a channel 430, an opening 441, a separator 442, and a channel 443. The hole 402 penetrates the substrate 401 at the center of the substrate 401. The container 400 is set in the sample measuring device 40 such that the center of the hole 402 coincides with a rotation axis 122, which will be described later. Hereinafter, the radial direction and the circumferential direction of a circle centered on the rotation axis 122 are respectively referred to as the "radial direction" and the "circumferential direction". Each of the chambers 421 to 426 is a space that can accommodate liquid. The chambers 421 to 426 are arranged in the circumferential direction near the outer periphery of the substrate 401.

The channel 430 comprises six radial regions 431 extending in the radial direction and an arc-shaped circumferential region 432 extending in the circumferential direction. The circumferential region 432 is connected to the six radial regions 431. The six radial regions 431 are respectively connected to the chambers 421 to 426. The six accommodating portions 411 are connected to the channel 430 via flow paths in the radial direction. The six accommodating portions 411 are respectively arranged side by side in the radial direction with the corresponding chambers 421 to 426. The accommodating portion 412 is connected via a flow path mainly extending in the radial direction to a flow path that connects the chamber 426 and the accommodating portion 411. A total of seven accommodating portions 411 and 412 are arranged on the inner peripheral side of the container 400, and a total of six chambers 421 to 426 are arranged on the outer peripheral side of the container 400.

Both the accommodating portion 411 and the accommodating portion 412 accommodate a reagent and comprise a sealing body 450 on the inner upper surface in the radial direction. The sealing body 450 is configured to be openable by being pressed from above by an opening part (not shown). Before the sealing body 450 is opened, the reagent inside the accommodating portion 411 does not flow into the channel 430, and when the sealing body 450 is opened, the reagent inside the accommodating portion 411 flows out into the channel 430. When the container 400 is rotated, the reagent moves to the corresponding chambers 421 to 426 due to centrifugal force.

A sample is injected into the opening 441. The sample is a whole blood sample collected from a subject. The blood sample is injected into the separator 442 via the opening 441. The separator 442 separates the injected blood sample into blood cells and plasma. The plasma separated by the separator 442 moves to the channel 443. When the container 400 is rotated, the plasma inside the channel 443 moves to the chamber 421 due to centrifugal force. As a result, a predetermined amount of plasma is transferred to the chamber 421.

Dried magnetic particles are fixed in the chamber 421. The sample measuring device 40 is configured to cause the magnetic particles to carry the analyte and the labeling material by sequentially transferring the magnetic particles to a plurality of chambers, and to detect the analyte based on the labeling material. That is, the magnetic particles carrying the analyte are moved in the radial direction by magnetic force. As a result, the magnetic particles are moved in the radial direction between the inside of the chamber 421 and the arc-shaped circumferential region 432 of the channel 430. When the container 400 is rotated, the magnetic particles move in the circumferential direction within the arc-shaped circumferential region 432. By a combination of radial movement due to the action of magnetic force and circumferential movement due to rotation, the magnetic particles carrying the analyte are moved to the chambers 421 to 426, and processing using a reagent is performed in each of the chambers 421 to 426. Finally, the magnetic particles carrying the analyte and the labeling material are moved to the chamber 426, and measurement is performed by the measurement unit 46 detecting the labeling material.

The reagent inside the container 400 reacts with the analyte in the sample and causes a change that allows the analyte to be measured directly or indirectly from the outside of the container 400. For example, the reagent luminesces according to the amount of the analyte. The luminescence is, for example, chemiluminescence or fluorescence. The reagent includes, for example, a labeling material that specifically binds to the analyte. The labeling material produces, for example, a signal that can be measured from the outside of the container 400. The labeling material includes a chemiluminescent substance or a fluorescent substance, and so on. Also, the reagent may be one that develops color according to the amount of the analyte or one that causes turbidity according to the amount of the analyte.

The measurement unit 46 performs the measurement of the analyte directly or indirectly by detecting a change caused by the reaction between the analyte in the sample and the reagent. Specifically, the measurement unit 46 optically measures the sample accommodated in the container 400. The measurement unit 46 includes a photodetector such as a photomultiplier tube, a phototube, or a photodiode, for example, when detecting luminescence. The measurement unit 46 includes a light source and a light receiving element when detecting fluorescence, color development, or turbidity. In a case where a myoglobin measurement reagent is used as the reagent, the measurement unit 46 prepares a measurement sample in which the myoglobin is labeled by the regent. The photodetector of the measurement unit 46 detects optical signal from the myoglobin contained in the measurement sample. The optical signal increases according to the concentration of myoglobin. The controller 41 converts the optical signal detected by the measurement unit 46 into concentration of myoglobin by using a calibration curve, thereby obtaining a measurement value indicative of concentration of myoglobin in the sample.

As the reagent, it is possible to use an immunoassay reagent kit including a first reagent that contains an antigen or an antibody capable of binding to a target substance and particles, a second reagent that contains particles and a silicone defoamer, and a third reagent that contains a labeled antigen or a labeled antibody capable of binding to the target substance, as disclosed in JP 2011-047788, for example. The target substance is not particularly limited as long as it is a substance detectable by immunoassay. That is, the target substance only needs to be capable of forming a complex containing the target substance and the antigen or the antibody on the particles by an antigen-antibody reaction, and may be myoglobin, for example.

Here, it is known that the blood myoglobin concentration is related to muscle fatigue and muscle damage because myoglobin flows out into the blood after high-intensity training or the like is performed and becomes a high value compared to normal times (for example, Cabinet Secretariat press release material: https://www.cas.go.jp/jp/houdou/pdf/20200519shiryo1.pdf).

In the present embodiment, the blood myoglobin concentration is measured using the sample measuring device 40, and the measured blood myoglobin concentration and the exercise load of the player are managed in association with each other. Hereinafter, the measured value of the blood myoglobin concentration is referred to as the "measured myoglobin value".

Furthermore, in the present embodiment, it takes a minimum of about 10 minutes (for example, 17 minutes) from the measurement start (for example, the timing when the sample is injected into the opening 441) until the measurement of the blood myoglobin concentration is completed, and it takes a minimum of about 10 minutes until the measured myoglobin value becomes a state in which it can be output.
The measured myoglobin value may be displayed (an example of output) on the display 43, for example, and may also be transmitted (an example of output) to an external device via the communication unit 44.

### <First Embodiment>

The first embodiment is an embodiment concerning providing a user with information related to the exercise load and measurement of myoglobin of a player, and providing a recommended training menu for the player.
The content of the first embodiment is similarly applicable to other embodiments and variations.

### <Functional Configuration>

### (1) Functional Configuration of the Server 10

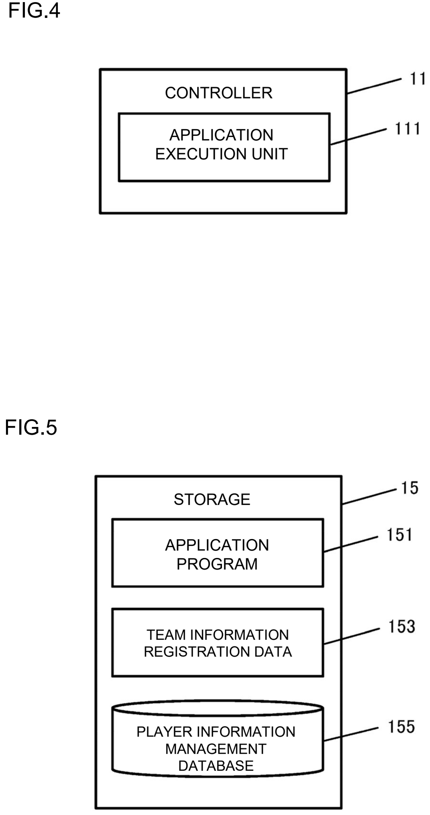

FIG. 4 is a diagram showing an example of a function realized by the controller 11 of the server 10 in the present embodiment.
The controller 11 comprises, for example, an application execution unit 111 that executes the application program 151 stored in the storage 15 as a functional unit.

FIG. 5 is a diagram showing an example of information stored in the storage 15 of the server 10 in the present embodiment.
The storage 15 stores, for example, an application program 151, team information registration data 153, and a player information management database 155.

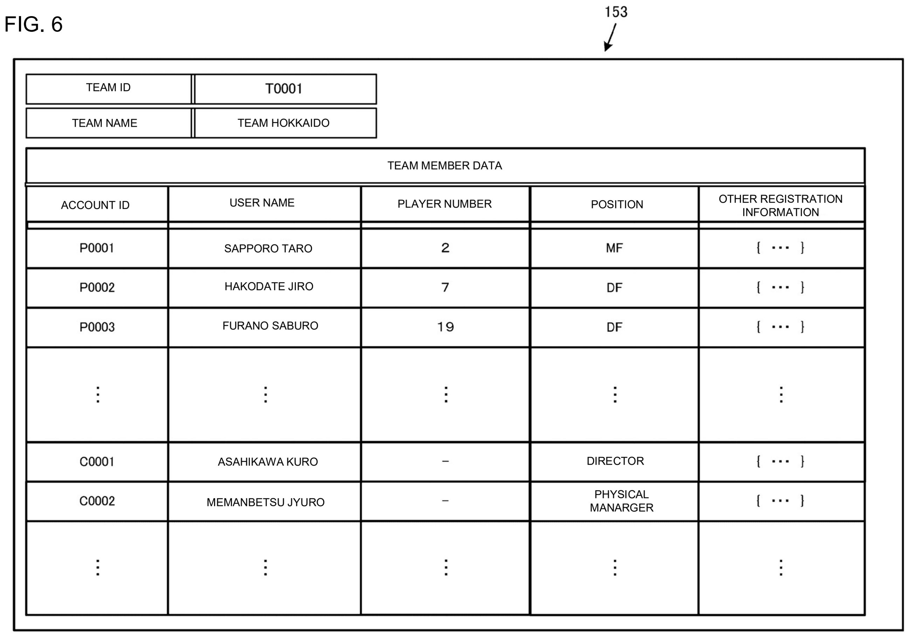

The team information registration data 153 is registration data concerning information related to a team and team members who are players belonging to the team (managers may be included, but basically players), and an example of its data configuration is shown in FIG. 6.
The team information registration data 153 stores, for example, a team ID which is identification information for identifying the team, the team name of the team, and team member data concerning the team members of the team.
The team ID is preferably a unique value for each team, and for example, a unique value (specific value) is set and stored by the server 10 for each team.
The team name is set by the server 10 as the name of the team input to the player terminal 20 or the manager terminal 30, for example.

The team member data stores, for example, an account ID, a user name of the team member with this account ID, information about the role of the user in the sport played by this team (for example, player number, position), and other registration information in association with each other.
The account ID is identification information for identifying this team member, is preferably a unique value for each account, and for example, a unique value (specific value) is set and stored by the server 10 for each account.
The user name is set by the server 10 as the name of the user input to the player terminal 20, for example.
The other registration information may store information such as user information (height, weight, age, etc.), identification information for identifying the player terminal 20 (IMEI (International Mobile Equipment Identity), etc.), the phone number of the player terminal 20 (terminal phone number), email address (terminal email address), and authentication information such as a password (login password, authentication password, etc.) used for various authentications in the application.

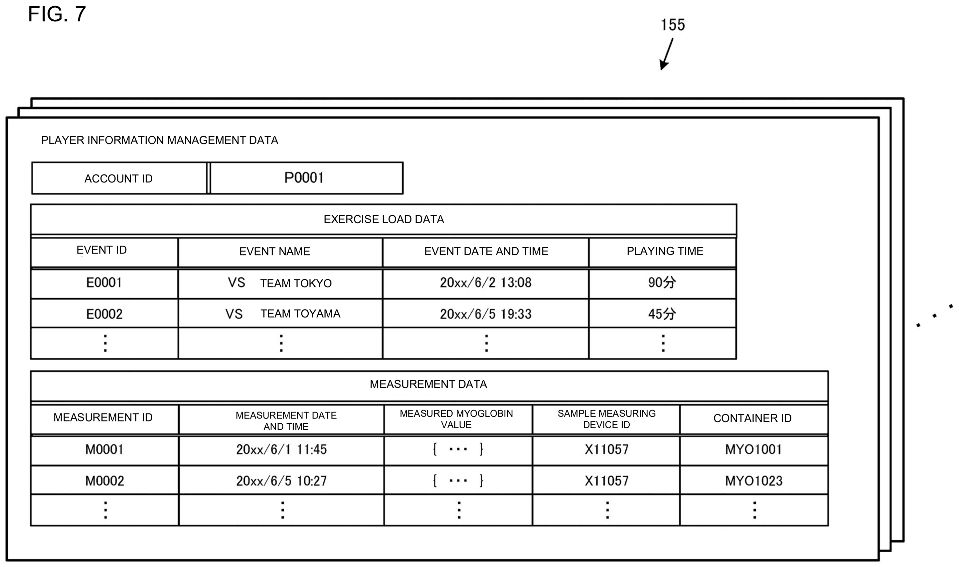

The player information management database 155 is data for managing information concerning the team member for each account of the team member stored in the team member data described above, for example, and an example of its data configuration is shown in FIG. 7.
The player information management database 155 stores player information management data as data for each account.
Each player information management data stores, for example, an account ID, exercise load data, and measurement data.

The exercise load data is data concerning the exercise load of the team member with this account ID, and for example, an event, an event name which is the name of this event, an event date and time which is the date and time when this event was held, and the playing time of this team member in this event are stored in association with each other.

The playing time is the time the player played in a game, for example (it may be the cumulative playing time of a plurality of games), and for example, the playing time for each player input to the player terminal 20 or the manager terminal 30 by a user, or the playing time for each player measured using the player terminal 20 or the manager terminal 30 may be transmitted to the server 10 and stored in the exercise load data of the corresponding player.

The playing time may be an example of the exercise load.
Furthermore, the playing time may be regarded as one of the elements of stats information related to fatigue (hereinafter referred to as "fatigue factor") among the stats information (information compiled as statistical information by breaking down the play of a player or a team and quantifying various items), and is one of the elements related to muscle fatigue (elements that can be considered to affect muscle fatigue). Such an element is conveniently referred to as a "muscle fatigue factor".

The measurement data is the measurement data of myoglobin measured by the sample measuring device 40 for the team member with this account ID, and for example, a measurement ID which is the identification information of the measurement, a measurement date and time, a measured myoglobin value, a sample measuring device ID which is the identification information of the sample measuring device 40, and a cartridge ID which is the identification information of the container 400 (cartridge) are stored in association with each other.

### (2) Functional Configuration of the Player Terminal

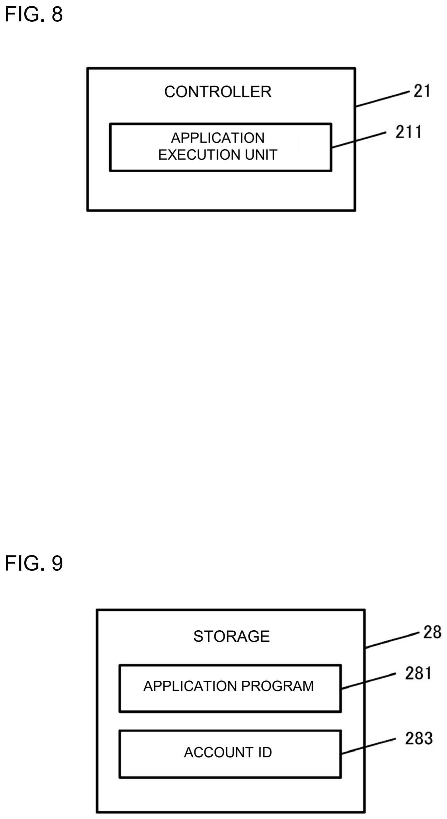

FIG. 8 is a diagram showing an example of a function realized by the controller 21 of the player terminal 20 in the present embodiment.
The controller 21 includes, for example, an application execution unit 211 that executes the application program 281 stored in the storage 28 as a functional unit.

FIG. 9 is a diagram showing an example of data and the like stored in the storage 28 of the player terminal 20 in the present embodiment.
The storage 28 stores, for example, an application program 281 and an account ID 283 which is the account of the player terminal 20 or the user of the player terminal 20.

Note that the manager terminal 30 may be the same, although the illustration is omitted.

### <Display Screen>

Hereinafter, examples of the display screen will be described. The following examples of the display screen may be screens displayed when the above application is executed on a terminal (player terminal 20, manager terminal 30), for example. Also, for clarity, some components are labeled with reference numerals on the display screen, but the reference numerals are not displayed on the actual display screen.

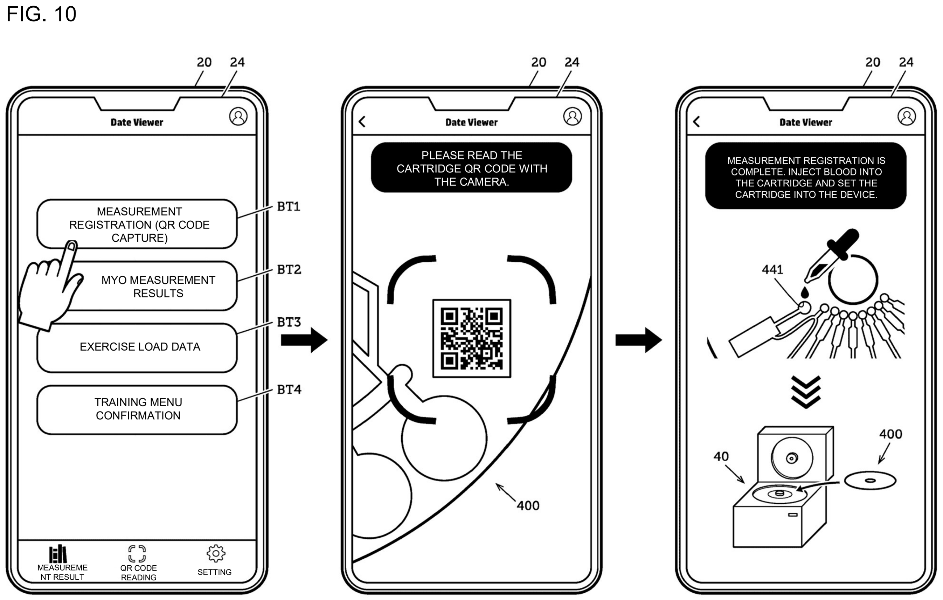

FIG. 10 is a diagram showing an example of a screen displayed on the display 24 of the player terminal 20.
As shown on the left side of FIG. 10, the player terminal 20 displays a screen including a button BT1 for activating a code reader for reading a two-dimensional code attached to a container 400 into which the blood sample of the player who is the user of the terminal is injected, a button BT2 for displaying the myoglobin measurement result, a button BT3 for displaying the exercise load data, and a button BT4 for displaying blood myoglobin and the recommended training menu for the player.

When the player taps the button BT1, the code reader is activated, and a guide frame and guidance for reading the two-dimensional code attached to the container 400 with a camera are displayed, as shown in the center of FIG. 10. When the player moves the player terminal 20 so that the two-dimensional code fits within the guide frame, the two-dimensional code is read, and identification information of the player terminal 20 and the player (for example, player ID, application ID, terminal ID) and the identification information of the container 400 (container ID, for example) acquired from the two-dimensional code are transmitted to the server 10, and both are stored in association with each other.

After the identification information of the container 400 and the identification information of the player are associated, information based on the completion of the association is transmitted from the server 10 to the player terminal 20, and the player terminal 20 that received the information displays a message urging the player to inject blood into the container 400 and set the container 400 in the sample measuring device 40, as shown on the right side of FIG. 10.

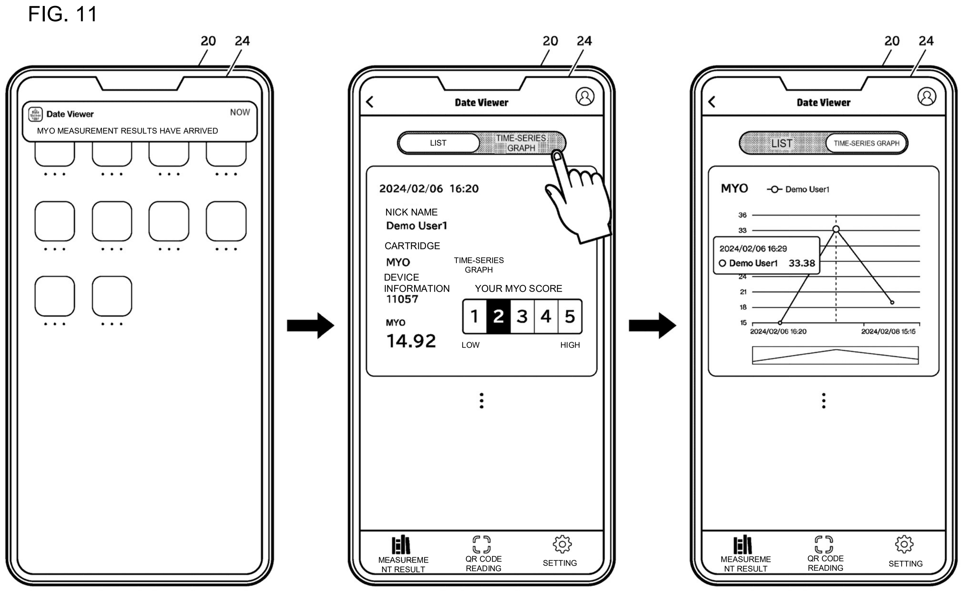

FIG. 11 is a diagram showing an example of information displayed on the display 24 of the player terminal 20 after the measurement of blood myoglobin is completed. As shown on the left side of FIG. 11, when the server 10 receives information based on the completion of the measurement of blood myoglobin from the sample measuring device 40 (including the identification information of the container 400), it sends a message notifying the player terminal 20 of the player to be measured (the player associated with the identification information of the container 400) that the measurement of blood myoglobin has been completed. For example, this message is received as a push notification on the player terminal 20.

By tapping this push notification, the player can check his/her blood myoglobin, as shown in the center of FIG. 11. In this example, the blood myoglobin is "14.92" and is shown to be the second lowest in a 5-level score. The player can understand that he/she is not in a state of relatively large muscle fatigue. Furthermore, by an operation, it is possible to display the time course of blood myoglobin in a graph format, as shown on the right side of FIG. 11. This makes it possible to confirm the situation where the blood myoglobin, although once greatly increased due to an event such as a game, is steadily decreasing due to subsequent rest.

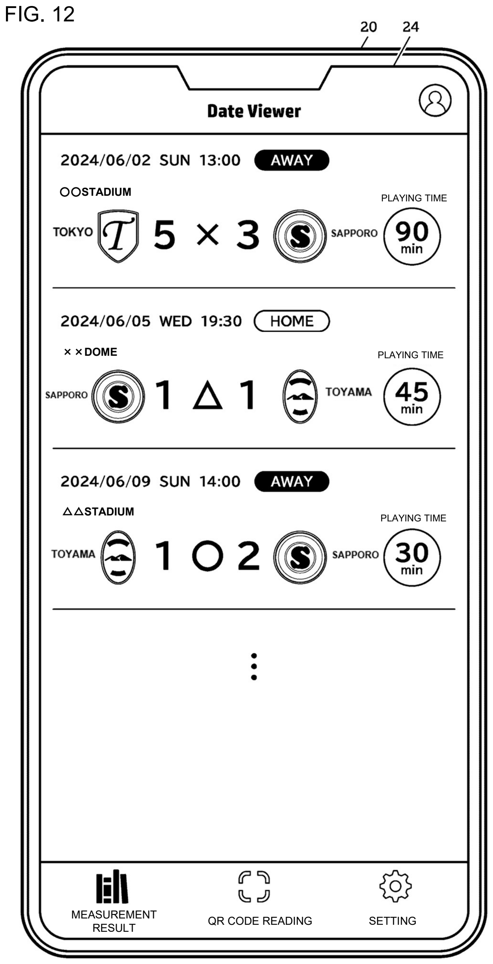

FIG. 12 shows an example in which the exercise load of each player is managed by the server 10 and the exercise load of the player is transmitted to the player terminal 20.
The display 24 of the player terminal 20 allows the player to check his/her playing time in each game (for example, 90 minutes for the game on June 2, 45 minutes for the game on June 5, and 30 minutes for the game on June 9). Therefore, the player can grasp both the muscle fatigue based on the blood myoglobin shown in FIG. 11 and the muscle fatigue in an event where high-intensity exercise such as a game occurs with his/her player terminal 20.

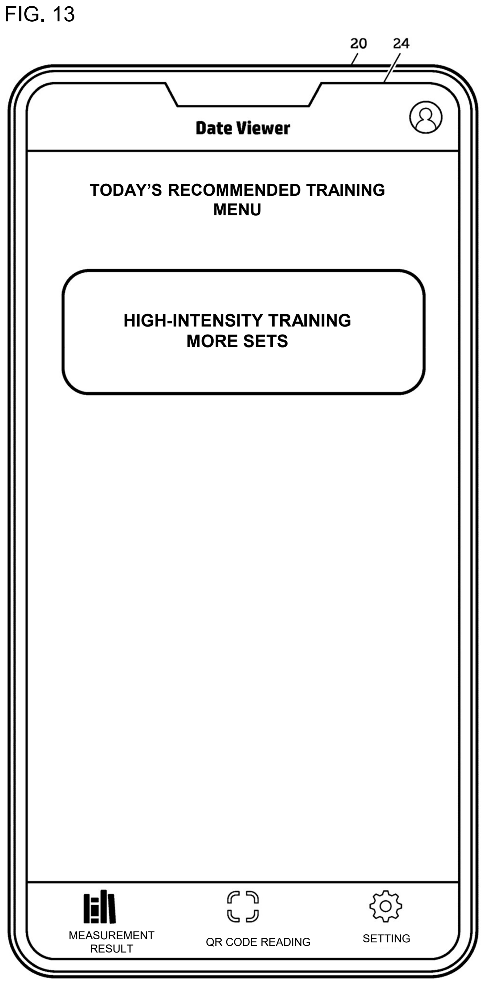

FIG. 13 is a diagram showing an example of a recommended training menu displayed on the player terminal 20.
The server 10 determines the recommended training menu for the player based on a method to be described later and transmits it to the player terminal 20. Note that it may be transmitted only to the player terminal 20 of the relevant player, or it may be transmitted to the player terminals 20 of players who are team members other than the relevant player. It may also be transmitted to the manager terminal 30. In this example, a high-intensity training menu (more sets) is determined as the recommended training menu based on the relatively low muscle fatigue of the player.

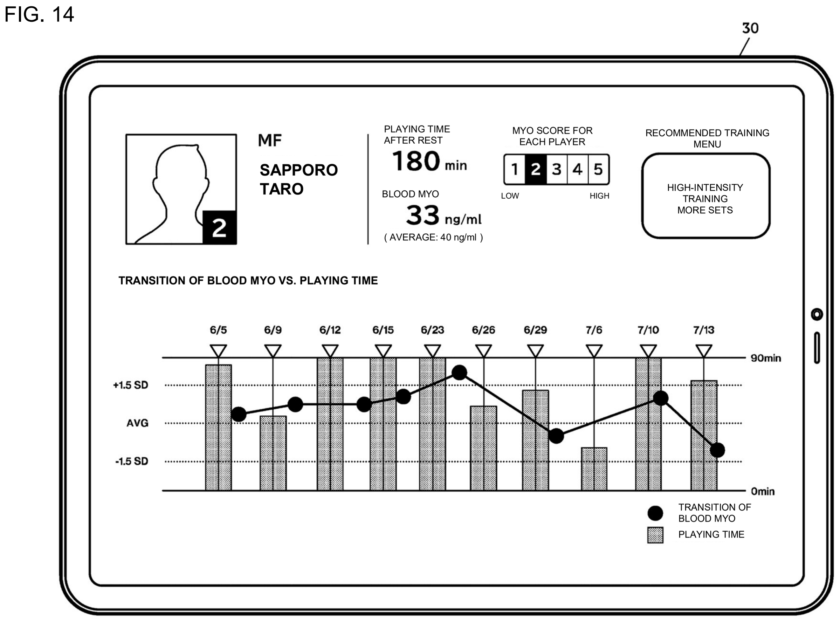

FIG. 14 is a diagram showing an example of a screen displayed on the manager terminal 30.
The manager terminal 30 can acquire (i) information related to muscle fatigue due to the player's participation in an event where muscle load occurs (for example, playing time in a game) and (ii) a measured myoglobin value measured at a predetermined timing (described later) from the information of each player managed by the server 10 for an arbitrary player, and display both on a common time axis.
This enables the manager to compare the relationship between both in a time series.

In this example, the playing time of a certain player in each of the past 10 games (6/5 to 7/13) and the transition of the measured myoglobin value measured after the game are displayed.
In this example, it is understood that the measured myoglobin value rapidly increased after the player played full-time (90 minutes) for three consecutive games (6/12, 6/15, 6/23).
For the subsequent two games (6/26, 6/29), as a result of not playing full-time and having a short playing time, it is understood that the measured myoglobin value has decreased to below the average value.

Furthermore, in this example, since the average measured myoglobin value of the player is 40 ng/ml and the latest measured myoglobin value is 33 ng/ml, the muscle fatigue is in a relatively low state, and the score of the measured myoglobin value is determined to be 2 out of 5 levels (second lowest), and high-intensity training is recommended.
In this way, since it is possible to grasp how the measured myoglobin value changes after an event that causes high-intensity load on the muscles, it becomes possible to control the participation status or playing time in the next event and to change the training menu or the like, according to the degree of muscle fatigue of each player.
It also becomes possible to perform management for the entire team so that the muscle fatigue of each player does not become excessive.

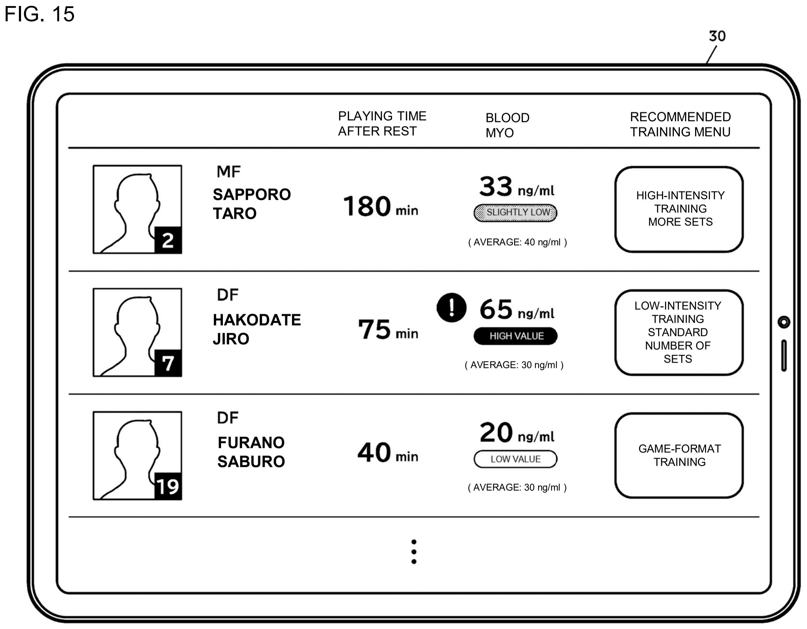

FIG. 15 is a diagram showing an example of information about a plurality of players displayed on the manager terminal 30. The manager terminal 30 acquires (i) information related to muscle fatigue due to the player's participation in an event where muscle load occurs (for example, playing time in a game), (ii) the latest measured myoglobin value, and (iii) the current recommended training menu for each of the plurality of players from the information of each player managed by the server 10, and displays them so that comparison between players is possible.

In this example, the measured myoglobin value for player No. 2 and player No. 19 is lower than the average (40 ng/ml and 30 ng/ml, respectively) (33 ng/ml, which is slightly low, and 20 ng/ml, which is a low value, respectively), but the measured myoglobin value for player No. 7 is extremely high (65 ng/ml) compared to the average (30 ng/ml), and a mark "!" indicating that the measured myoglobin value is higher than the average is displayed. Note that this mark may be displayed for a player whose measured myoglobin value is 4 or higher out of 5 levels, or this mark may be displayed only for a player whose measured myoglobin value is 5 out of 5 levels.
In this way, by displaying information that notifies the manager that the player requires attention in muscle fatigue management, the manager can appropriately grasp the player who requires attention, control the participation status or playing time in the next event, and change the training menu or the like.

Note that in the present embodiment, the controller 11 of the server 10 automatically determines the recommended training menu for each player in a recommended training menu determination process to be described later, but the present invention is not limited to this, and for example, a different training menu from the recommended training menu automatically determined by the server 10 may be set by the manager operating the manager terminal 30.

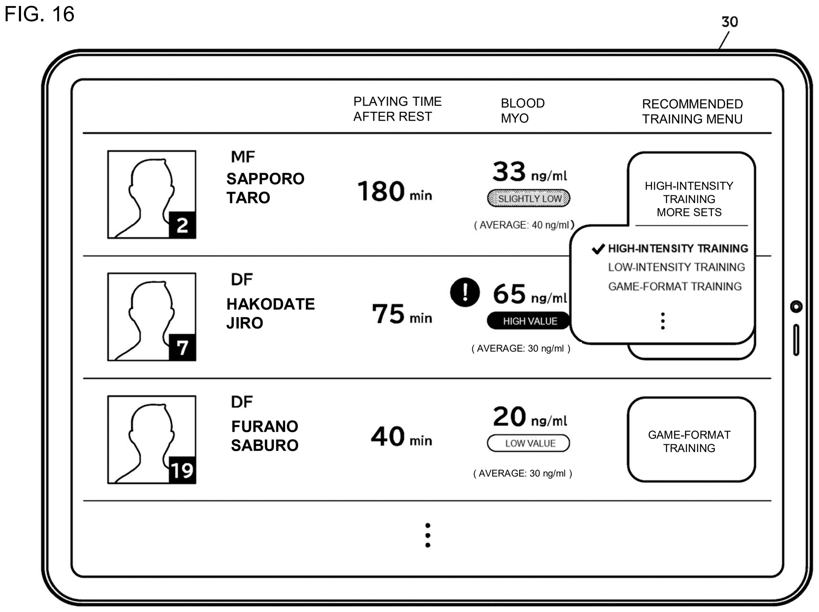

For example, as shown in FIG. 16 (a description of the same parts as in FIG. 15 is omitted), in addition to the training menu that the controller 11 of the server 10 can determine as the recommended training menu, it is possible to edit and newly set an arbitrary training menu by the input device of the manager terminal 30, such as a unique training menu like "high-intensity training only for a certain part (for example, abdominal muscles and back muscles) and low-intensity training for other parts."
The newly set training menu is transmitted to the server 10, stored in the storage 15 in association with the account ID, and transmitted to the player terminal 20 of the corresponding player (for example, player No. 2 in the case of FIG. 16), and the player performs training according to the newly set training menu.

The new training menu set on the manager terminal 30 may be transmitted to the server 10 and stored (i.e., newly registered) as a new training menu selectable by the manager terminal 30 or the like.
Note that the new training menu may be adopted (set and registered) as a training menu that can be automatically determined by the controller 11. For example, for a player for whom a new training menu is set by the manager, the conditions at that time (playing time, measured myoglobin value, score, etc.) may be stored, and subsequently, when the conditions are met (it may be only the same player, or different players may be included), the newly adopted training menu may be automatically determined and recommended by the controller 11.

In this way, based on the new training menu input in the manager terminal 30, the new training menu and the player's information at that time (playing time, measured myoglobin value, score, etc.) are associated, and the new training menu may be recommendable to subsequent players based on the player's information (playing time, measured myoglobin value, score, etc.) acquired thereafter.

### <Process>

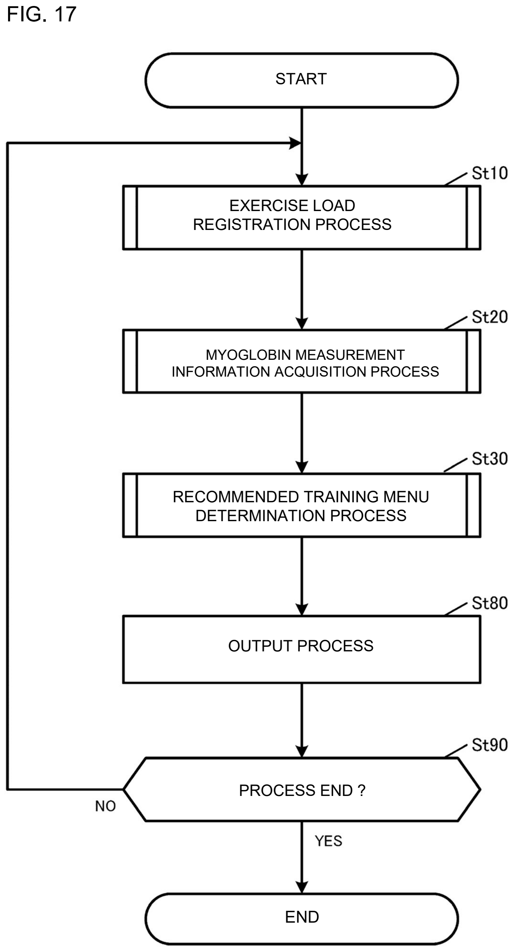

FIG. 17 is a flowchart showing an example of a flow of a process executed by the system 1 (each device of the system 1) in the present embodiment.
Note that the process of this flowchart is merely an example and is not limited to this. Also, another step may be added, or a part of the steps may be omitted (deleted).
For example, the order of Step St10 and Step St20 may be reversed.

First, an exercise load registration process is performed (St10), for example, between the server 10 and the manager terminal 30. Details will be described later.

Next, the controller 11 of the server 10 performs a myoglobin measurement information acquisition process (St20). Specifically, the measurement information of myoglobin is acquired and stored in the measurement data in the player information management data corresponding to each player (for example, each team member). Details will be described later.

Thereafter, the controller 11 of the server 10 performs a recommended training menu determination process (St30) that determines the recommended training menu. In this process, the recommended training menu to be recommended to the player is determined for each player, for example. Details will be described later.

Next, the controller 11 of the server 10, the controller 21 of the player terminal 20, the controller 31 of the manager terminal 30, and the like perform an output process (St80) that outputs various information, for example.
In this case, for example, the server 10 transmits various information to the terminal (player terminal 20 or manager terminal 30) in response to a request from the terminal, and the terminal may notify the user of the terminal of the information received from the server 10 by display or the like.

The information to be output may be at least one of the following information, for example:
- Information related to the exercise load for each player
- Information related to the measurement of myoglobin for each player
- Recommended training menu for each player

The information related to the exercise load for each player may include, for example, the exercise load for each player registered in St10 (playing time in the present embodiment) and information such as the event ID, event name, and event date and time associated therewith.

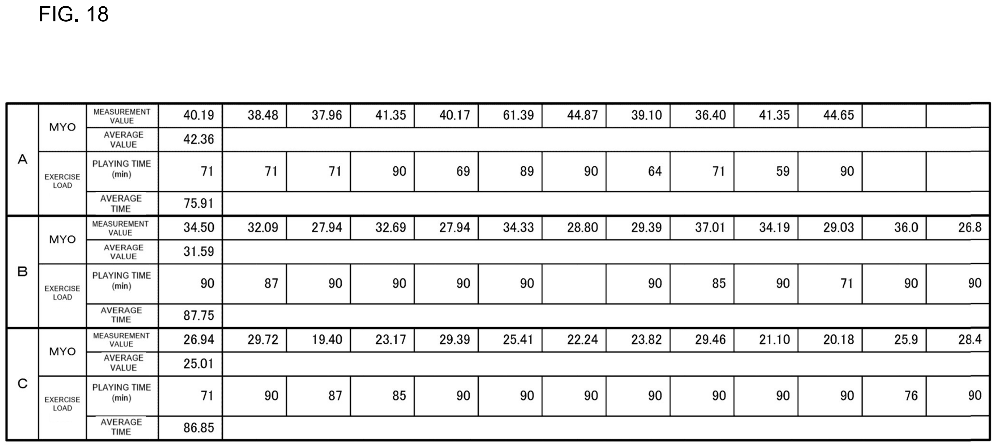

The information related to the measurement of myoglobin for each player may include, for example, the measured myoglobin value for each player registered in St20 and the measurement ID, measurement date and time, and the like associated therewith. The sample measuring device ID and cartridge ID and the like may be included. Also, a reference value (for example, average value) and standard deviation of myoglobin for each player may be included.
As an example of the reference value of myoglobin for each player, the results of myoglobin measurement and playing time performed multiple times by three players are shown in FIG. 18. In this figure, the measured myoglobin value is indicated as MYO.

From this figure, it is understood that the average values of the measured myoglobin values of the three players (Player A to Player C) are different: Player A is "42.36", Player B is "31.59", and Player C is "25.01". Despite the fact that all of these players are players who play close to full-time, the average values of the measured myoglobin values are largely different, and the individual differences are large. Therefore, it is preferable to set the reference value for evaluating myoglobin for each player.

The measurement for setting the reference value of myoglobin is preferably performed after a predetermined time has elapsed from the game. The value of myoglobin rapidly increases immediately after the game, then gradually decreases, and returns to the normal value in approximately 72 hours. Therefore, in the present embodiment, for example, where the training menu after rest is determined, the reference value of myoglobin can be leveled by using the predetermined time of 72 hours.
Also, the measured myoglobin value taken within a certain time immediately after the game (for example, within 24 hours) can be used as the reference value for myoglobin. In that case, the reference value of myoglobin in a state of high muscle fatigue can be calculated.

Furthermore, if there is a player whose measured myoglobin value is higher than a predetermined value, alert information warning that the myoglobin for the player is high may be included in the information related to the measurement of myoglobin for each player, and the alert may be output by the server 10, player terminal 20, manager terminal 30, or the like.

Note that the output of information may include display of information by a display device (for example, display of information on a server or terminal), sound output of information by a sound output device (for example, sound output of information on a server or terminal), and transmission of information to an external device (for example, transmission of information from a server to a terminal).

Thereafter, the controllers of the various devices constituting the system 1 determine whether or not to terminate the process (St90), and if it is determined to continue the process (St90: No), the process returns to St10, for example. On the other hand, if it is determined to terminate the process (St90: Yes), the process terminates.

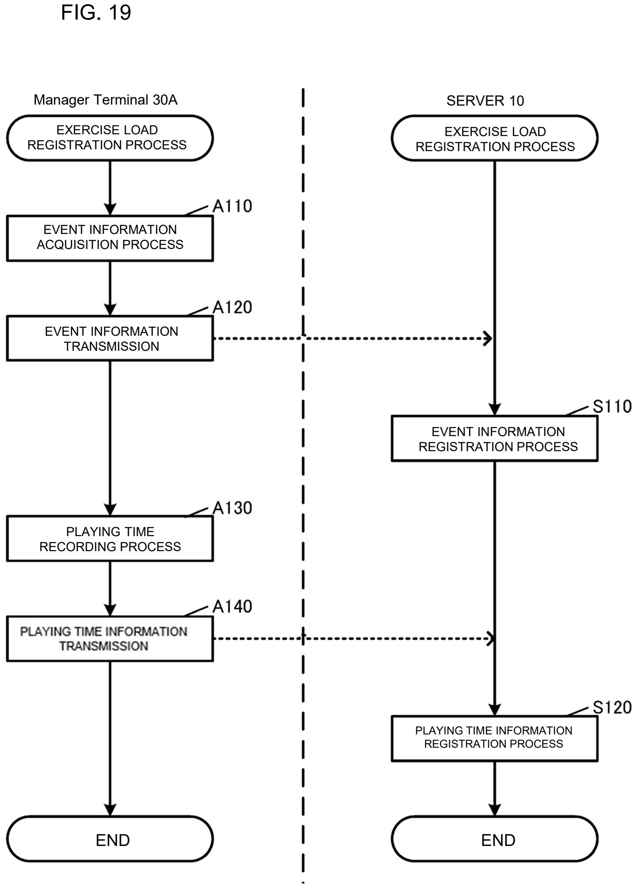

FIG. 19 is a flowchart showing an example of the flow of the exercise load registration process in St10 of FIG. 17. In this flowchart, the process performed by the controller 31 of the manager terminal 30A, which is one of the manager terminals 30, is shown on the left side, and the process performed by the controller 11 of the server 10 is shown on the right side.
Note that the process of this flowchart is merely an example and is not limited to this. Also, another step may be added, or a part of the steps may be omitted (deleted).

The controller 31 of the manager terminal 30A performs an event information acquisition process (A110) that acquires event information based on user input to the terminal or the like, and transmits the acquired event information to the server 10 (A120). When the server 10 receives the event information from the manager terminal 30A, the controller 11 of the server 10 performs an event information registration process (S110). Specifically, the playing time of the corresponding player is stored in the exercise load data in the player information management data corresponding to each player (team member) belonging to the team of the user of the manager terminal 30A, in association with the event ID, event name, and event date and time.
Note that the information of the player may be received, acquired, and registered by the server 10 from the manager terminal 30A or the player terminal 20 separately from the present process, or the manager terminal 30A may include it in the event information and transmit it to the server 10, and the server 10 may acquire and register the information of the player from the event information.

After A120, the controller 31 of the manager terminal 30A performs a playing time recording process (A130) that records the playing time in the event for each player, and transmits the recorded playing time information to the server 10 (A140).
Then, the controller 31 of the manager terminal 30 terminates the exercise load registration process.

When the server 10 receives the playing time information, the controller 11 of the server 10 performs a playing time information registration process (S120). Specifically, the playing time of the corresponding player is stored in the record where the event ID is stored in the exercise load data corresponding to each of the players.
Then, the controller 11 of the server 10 terminates the exercise load registration process."

Note that in the present process, the server 10 received and acquired the event information and the playing time information from the manager terminal 30, but they may be received and acquired from the player terminal 20.
Also, for example, the server 10 may acquire (automatically import, for example) stats information that is automatically generated by a Bot or the like and is disclosed on the Internet by a predetermined operator.

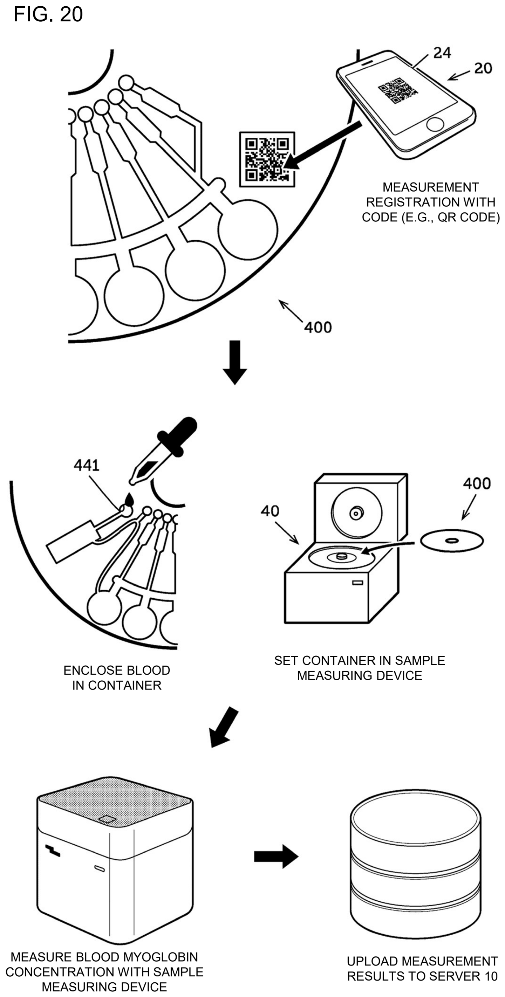

FIG. 20 is a diagram illustrating a method for acquiring and recording myoglobin measurement information in the myoglobin measurement information acquisition process of St20 in FIG. 17.
For example, the player starts the application on his/her player terminal 20, logs in, selects "Code Reading" from the measurement registration functions of the application, and activates the code reader. Then, the user causes the code reader to read the two-dimensional code of the container 400. The player terminal 20 acquires the cartridge ID, which is the identification information of the container 400 (cartridge), from the reading result by decoding. Then, the player terminal 20 transmits the acquired cartridge ID to the server 10.
The controller 11 of the server 10 stores the received cartridge ID in association with the set measurement ID in the measurement data in the player information management data corresponding to the account ID of the user of the player terminal 20.
When the measurement by the sample measuring device 40 is completed, for example, the sample measuring device 40 transmits the cartridge ID, the sample measuring device ID which is the identification information of its own device, the measurement date and time, and the measured myoglobin value to the server 10.
If the cartridge ID stored in the measurement data and the cartridge ID received from the sample measuring device 40 are the same, the controller 11 of the server 10 stores the measurement date and time, the measured myoglobin value, and the sample measuring device ID received from the sample measuring device 40 in association with each other in the record of the measurement ID.
Note that the server 10 may transmit (push transmit) the information of the myoglobin measurement result to the player terminal 20 in the form of a push notification or the like, and the player terminal 20 may display the received push notification on the display 24.

Note that the above method is merely an example and is not limited to this.

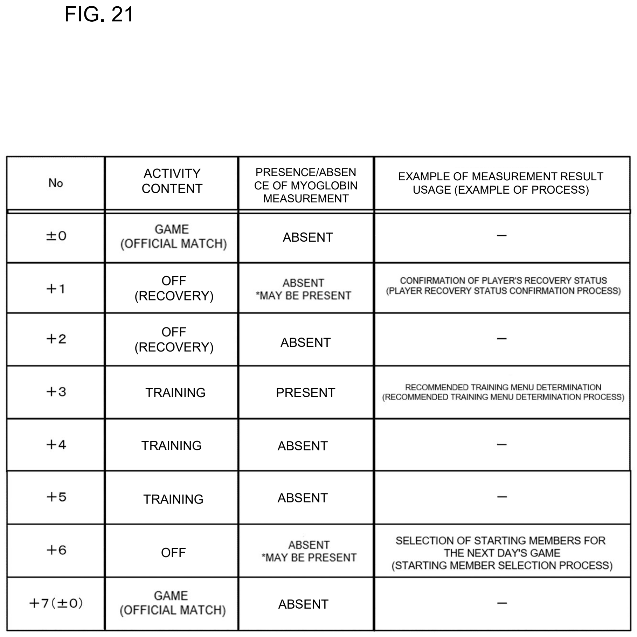

FIG. 21 is a diagram illustrating the myoglobin measurement timing.
This example shows a table where it is the principle that a game (a regular game in this example) is held once a week, and one week is one cycle. In this table, for example, the number (No) for each day in one cycle is shown by adding 1 with the day on which the game is held as "±0".
For the number, the content of the player's activity on that day, myoglobin measurement presence/absence which is information indicating whether or not myoglobin measurement is performed, and a usage example of the measurement result which is an example of the myoglobin measurement result usage, are defined.

In this example, "Yes" is defined for myoglobin measurement presence/absence only on the training day with number "+3". Specifically, the two days from the day of the game are off for the player's recovery, and training is performed from the next day, but myoglobin measurement is stipulated to be performed on the first day of the training.

Furthermore, among the usage examples of the measurement result, "Recommended Training Menu Determination" is defined as the usage example of the measurement result on the training day with number "+3" on which myoglobin measurement is performed.
This may be realized by the process (recommended training menu determination process) of Step St30 in the flowchart of FIG. 17, and details will be described later.

Note that in this example, myoglobin measurement is performed only one day a week, but it is not limited to this, and for example, the myoglobin measurement may be performed on the day with number "+1", which is the off day after the game. A usage example of the measurement result in this case is, for example, "Confirmation of the player's recovery level". That is, it is conceivable to confirm the recovery level from fatigue from the game for each player based on the measured myoglobin value of each player on this measurement day.
In this case, for example, the controller 11 of the server 10 may perform a corresponding process (player recovery level confirmation process).

Also, for example, the myoglobin measurement may be performed on the day with number "+6", which is the off day before the game.
A usage example of the measurement result in this case is, for example, "Selection of the starting members for the game the next day". That is, it is conceivable to select the players to be used as starting members for the game the next day based on the measured myoglobin value of each player on this measurement day.
In this case, for example, the controller 11 of the server 10 may perform a corresponding process (starting member selection process).

Note that the day with number "+3" may be a game day (two games a week), and in this case, the myoglobin measurement may not be performed on the game day. Also, if there was a game on the day with number "±0" or "+3", the cumulative playing time of the game may be used. Also, the myoglobin measurement may be performed after the game.

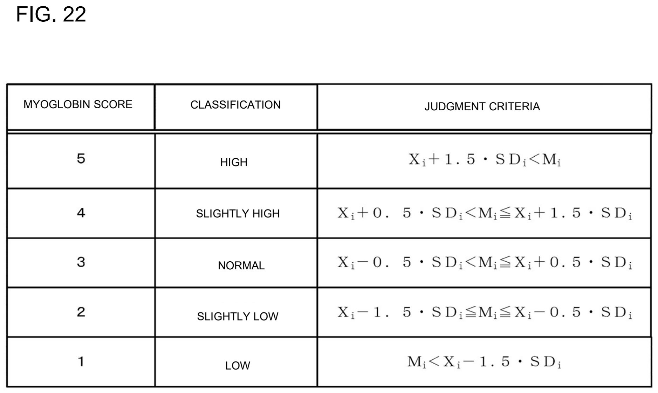

FIG. 22 is a diagram illustrating the calculation method of the myoglobin score used in the recommended training menu determination process of St30 in FIG. 17. This figure shows a table when "1 to 5" is set as the myoglobin score, and the myoglobin score, category, and determination condition are defined in association with each other.
The myoglobin score may be a score determined for each player based on the determination condition, for example, and this is referred to as the myoglobin score for each player.

Hereinafter, the measured myoglobin value to be determined for each player "I" (i=1, 2, ...) is denoted as "Mi".
This measured myoglobin value "Mi" to be determined may be the measured myoglobin value of the player "i" measured at the measurement timing of FIG. 21 described above, for example.

Furthermore, the average value of the measured myoglobin values of each player "i" from multiple past measurements is denoted as "Xi", and the standard deviation is denoted as "SDi". These values may be calculated by the server 10 based on the measured myoglobin values measured after a predetermined time (for example, 48 hours from the end of the game) has elapsed from the game, and measured a predetermined number of times (for example, 10 times). That is, the measured myoglobin value measured at a timing when the player's muscle fatigue is considered to have recovered after the game may be used.

The myoglobin score "5" has a category of "High" (highest muscle fatigue), and the determination condition is defined as "Xi+1.5·SDi<Mi".
The myoglobin score "4" has a category of "Slightly High" (second highest muscle fatigue), and the determination condition is defined as "Xi+0.5·SDi<Mi≤Xi+1.5·SDi". The myoglobin score "3" has a category of "Normal" (standard level of muscle fatigue), and the determination condition is defined as "Xi-0.5·SDi<Mi≤Xi+0.5·SDi".
The myoglobin score "2" has a category of "Slightly Low" (second lowest muscle fatigue), and the determination condition is defined as "Xi-1.5·SDi≤Mi≤Xi-0.5·SDi".

The myoglobin score "1" has a category of "Low" (lowest muscle fatigue), and the determination condition is defined as "Mi<Xi-1.5·SDi".

The myoglobin score determined for each player in this way becomes a score based on the reference value based on the measured myoglobin values measured multiple times for each player, and thus becomes a score that reflects the individual differences of the player.

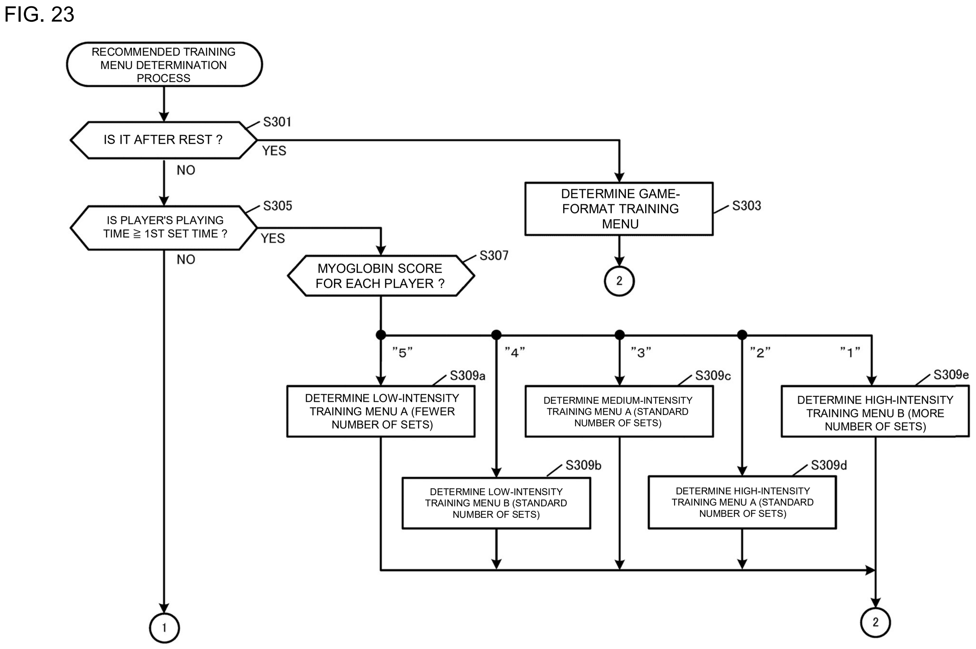

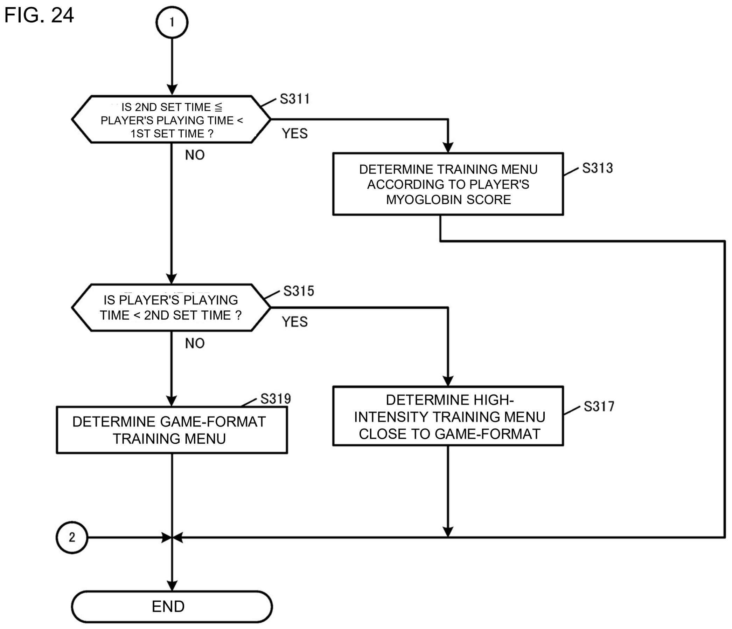

FIGS. 23 and 24 are flowcharts showing an example of the flow of the recommended training menu determination process in St30 of FIG. 17.
The controller 11 of the server 10 determines whether or not the player is returning from rest (S301). Specifically, it determines that the player is returning from rest when a predetermined time (for example, 72 hours) has elapsed since the most recent game was completed, for example.

When it is determined that the player has not recovered from a rest period (S301: NO), the controller 11 of the server 10 determines whether or not the playing time of the past most recent game of the player is equal to or greater than a first set time (e.g., 180 minutes) (S305). The playing time in this case may be, for example, the cumulative time during which the player has played in a game since the player took a rest and the myoglobin score became equal to or less than the average (the myoglobin score may be "1").

When it is determined that this condition is satisfied (S305: YES), the controller 11 of the server 10 determines the myoglobin score of the player (S307). When the myoglobin score is "5" (S307: "5"), the controller 11 of the server 10 determines a low-intensity training menu A (smaller number of sets) as the recommended training menu for the player (S309a). When the myoglobin score is "4" (S307: "4"), the controller 11 of the server 10 determines a low-intensity training menu B (standard number of sets) as the recommended training menu for the player (S309b). When the myoglobin score is "3" (S307: "3"), the controller 11 of the server 10 determines a medium-intensity training menu A (standard number of sets) as the recommended training menu for the player (S309c). When the myoglobin score is "2" (S307: "2"), the controller 11 of the server 10 determines a high-intensity training menu A (standard number of sets) as the recommended training menu for the player (S309d). When the myoglobin score is "1" (S307: "1"), the controller 11 of the server 10 determines a high-intensity training menu B (larger number of sets) as the recommended training menu for the player (S309e). As an approach, for example, a training menu with higher intensity (load) may be determined as the recommended training menu as the myoglobin score is lower.

In this example, the low-intensity training menu may be, for example, a menu that restricts or prohibits high-intensity (high load) training such as high-speed running or player-vs-player training. For example, it may be a menu that limits the high-speed running at 20 km/h or more to 150 m or less, or a menu that prohibits the player-vs-player training. The high-intensity training menu may be, for example, a menu that permits the high-intensity training described above. For example, it may be a menu that permits the high-speed running at 20 km/h or more for 300 m or more, or a menu that permits the player-vs-player training. The medium-intensity training menu may be a training menu with a load intermediate between the low-intensity training menu and the high-intensity training menu.

Note that the myoglobin score for each player is a score reflecting the individual difference of the player as described above. Therefore, a training menu that considers the individual difference of the player is determined as the recommended training menu for the player.

After these, the controller 11 of the server 10 finishes the recommended training menu determination process.

When it is determined that the condition of S305 is not satisfied (S305: NO), the controller 11 of the server 10 determines whether or not the playing time of the player is equal to or greater than a second set time (e.g., 90 minutes) and less than the first set time (e.g., 180 minutes) (S311).

When it is determined that this condition is satisfied (S311: YES), the controller 11 of the server 10 determines a training menu corresponding to the myoglobin score of the player as the recommended training menu for the player (S313). Specifically, for example, the following may be set: · Myoglobin score "5": Low-intensity training menu B (standard number of sets) · Myoglobin score "4": Medium-intensity training menu A (standard number of sets) · Myoglobin score "3": High-intensity training menu A (standard number of sets) · Myoglobin score "2": High-intensity training menu B (larger number of sets) · Myoglobin score "1": High-intensity training menu C (larger number of sets + α). As an approach, for example, a training menu with higher intensity (load) compared to the training menu determined in S309 (S309a to S309e) for the same myoglobin score may be determined as the recommended training menu.

Then, the controller 11 of the server 10 finishes the recommended training menu determination process.

When it is determined that the condition of S311 is not satisfied (S311: NO), the controller 11 of the server 10 determines whether or not the playing time of the player is less than the second set time (e.g., 90 or more) (S315).

When it is determined that this condition is satisfied (S315: YES), the controller 11 of the server 10 determines, for example, a high-intensity training menu close to a game format as the recommended training menu for the player (S317). As an approach, for example, a training menu with higher intensity (load) compared to the training menu determined in S313 may be determined as the recommended training menu.

Then, the controller 11 of the server 10 finishes the recommended training menu determination process.

When it is determined in S310 that the player has recovered from a rest period (S301: YES), or when it is determined that the condition of S315 is not satisfied (S315: NO), the controller 11 of the server 10 determines, for example, a game-format training menu (the training menu with the highest intensity) as the recommended training menu for the player (S319). As an approach, for example, a training menu with higher intensity (load) compared to the training menu determined in S317 may be determined as the recommended training menu.

Note that the game-format training menu in this example may be, for example, the training menu with the highest intensity (load), but some restrictions may be imposed so that the load is not excessive, for example, by prohibiting running at a speed of 90% or more of each individual player, or at a speed of 30 km/h or more.

Then, the controller 11 of the server 10 finishes the recommended training menu determination process.

Note that, as described above, an output process for outputting information such as information on the exercise load for each player and information on the measurement of myoglobin for each player may be performed without performing the recommended training menu determination process (St30). That is, the process of determining the recommended training menu may be omitted. For example, by outputting at least the exercise load of the player and the measured myoglobin value of the player in response to a request from the player terminal 20 or the manager terminal 30, the player and the manager can check the exercise load and the measured myoglobin value of the player, and these pieces of information can be used for the physical condition management (health management) of the player.

<Effect of First Embodiment> In the present embodiment, a system (e.g., system 1) for supporting physical condition management (health management) of an athletic player includes a measurement device (e.g., sample measuring device 40) for measuring blood myoglobin contained in the blood of the player, and a server (e.g., server 10) for recording the exercise load of the player and the measured value of the blood myoglobin. The server provides the exercise load of the player and the measured value of the myoglobin in response to a request from a terminal (e.g., player terminal 20, manager terminal 30). This allows the exercise load and the measured value of myoglobin of the athletic player to be provided in response to a request from the terminal, and the physical condition management (health management) of the player can be supported. Further, a user can check the exercise load and the measured value of myoglobin of the player on the terminal.

Furthermore, the server generates a reference value of myoglobin for each player based on a plurality of times the measured myoglobin value has been measured (e.g., the average value of myoglobin for each player), and evaluates a newly measured value of myoglobin based on the reference value. By evaluating the newly measured myoglobin value based on the reference value of myoglobin generated for each player based on the myoglobin value measured a plurality of times, the measured myoglobin value of can be appropriately evaluated considering the individual difference of the player.

In this case, the server may calculate the reference value based on the measured value of myoglobin measured after a predetermined time has elapsed from a game. This allows an appropriate value to be calculated as the reference value of myoglobin, for example, based on the measured value of myoglobin in a state where the muscle fatigue of the player due to the game has been alleviated to some extent.

Furthermore, the server may transmit an evaluation result (e.g., myoglobin score for each player) based on the measured value of myoglobin and the reference value of myoglobin to a terminal (e.g., player terminal 20) associated with the account of the player. This allows the evaluation result based on the measured value of myoglobin and the reference value of myoglobin to be informed to the player.

Furthermore, the measurement device (e.g., sample measuring device 40) may measure myoglobin by mixing a reagent including a labeling antibody with the blood to label the myoglobin with the labeling antibody, and optically detecting the labeled myoglobin. This allows myoglobin to be appropriately measured.

Furthermore, the measurement device (e.g., sample measuring device 40) may separate the blood into a plasma component and a blood cell component, and mix the plasma component with the reagent mentioned above. This allows the plasma component and the reagent including the labeling antibody to be mixed to label the myoglobin with the labeling antibody.

Furthermore, the measurement device (e.g., sample measuring device 40) may output the measured value after a predetermined time has elapsed from the measurement start. This allows the measured value of myoglobin to be acquired after a predetermined time has elapsed from the measurement start.

Furthermore, an alert may be output for a player whose measured value of myoglobin is higher than a predetermined value. This allows a warning to be issued to the player by outputting an alert for the player whose measured value of myoglobin is a high value.

Furthermore, the server may determine a recommended training menu (e.g., recommended training menu) based on the exercise load and the measured value of myoglobin, and output the training menu to a terminal (e.g., player terminal 20, manager terminal 30). This allows the recommended training menu to be appropriately determined based on the exercise load and the measured value of myoglobin. In addition, by outputting the determined training menu to the terminal, the player or the manager can be informed.

<Modification of First Embodiment> In the above-described embodiment, playing time was illustrated as the exercise load of the player, but the present invention is not limited to this. Muscle fatigue elements other than playing time may be used, for example, muscle fatigue elements such as the player's "running distance", "maximum running speed", "sprint" (e.g., the number of times the player ran at 25.0 km/h or more for 1 second or more), and "number of turns" may be used. These pieces of information may be acquired by the server 10, for example, using the same method as the method described in the first embodiment. Note that the information may be acquired from a wearable device worn by the player, although details will be described later.

For example, when the recommended training menu is determined with the playing time described in the above-described embodiment being essential, the controller 11 of the server 10 may determine a training menu with the intensity (load) lowered by one level compared to the training menu determined in the processes of FIGS. 23 and 24, for example, as the recommended training menu for the player, when at least one of the running distance, maximum running speed, sprint, and number of turns of the player is equal to or greater than (or exceeds) a predetermined threshold. By doing this, even if the playing time of the player was short, if it is estimated that the exercise load of the player was high, the load of the training of the player can be reduced.

Conversely, the controller 11 of the server 10 may determine a training menu with the intensity (load) raised by one level compared to the training menu determined in the processes of FIGS. 23 and 24, for example, as the recommended training for the player, when at least one of the running distance, maximum running speed, sprint, and number of turns of the player is less than (or equal to) a predetermined threshold. By doing this, even if the playing time of the player was long, if it is estimated that the exercise load of the player was low, the load of the training of the player can be increased.

<Second Embodiment> A second embodiment is an embodiment related to acquiring an index value indicating the degree of muscle fatigue of a player and outputting information based on the acquired index value. The content of the second embodiment can be similarly applied to other embodiments and modifications.

In this embodiment, for example, the controller 11 of the server 10 calculates the muscle fatigue level of the player based on information regarding the measurement of myoglobin and the muscle fatigue factor. That is, the muscle fatigue level of the player is calculated based on the myoglobin measurement information and the exercise load related to the muscle fatigue of the player. Specifically, for example, the product of the myoglobin score of the player and the muscle fatigue factor of the player is calculated as the muscle fatigue level of the player for one player.

Furthermore, for example, the sum of the value obtained by multiplying the myoglobin score of the player by a coefficient and the value obtained by multiplying the muscle fatigue factor of the player by a coefficient may be calculated as the muscle fatigue level of the player for one player. Alternatively, for example, the muscle fatigue level (objective variable) of the player may be calculated based on a learned artificial intelligence model which is a multivariate artificial intelligence model that uses the myoglobin score of the player and the muscle fatigue factor of the player as explanatory variables for one player.

In this embodiment, for example, the controller 11 of the server 10 performs a threshold determination on the muscle fatigue level for each player calculated as described above, and determines the recommended training menu for each player. Specifically, for example, a training menu with lower intensity (load) is determined as the recommended training menu for the player as the muscle fatigue level of the player is higher.

In this case, for example, the player may measure blood myoglobin at a predetermined measurement timing as the timing for measuring blood myoglobin to calculate the muscle fatigue level. This measurement timing may be, for example, the timing corresponding to No. "+3" in FIG. 21 (myoglobin measurement presence/absence is "present"), and the recommended training menu for the player may be determined based on the muscle fatigue level based on the measurement result at this measurement timing.

When the playing time of the player was long (e.g., 180 minutes or more as previously described), it may be considered that recommending a high-intensity training menu to the player is undesirable even if the blood myoglobin was low. However, some players may have a high recovery ability from muscle fatigue and may perform high-intensity training menus from an early stage. By using an index value (e.g., muscle fatigue level) in which both the myoglobin measurement information and the exercise load measurement information are taken into account, instead of using them independently as described above, a more flexible proposal of a recommended training menu for each player becomes possible.

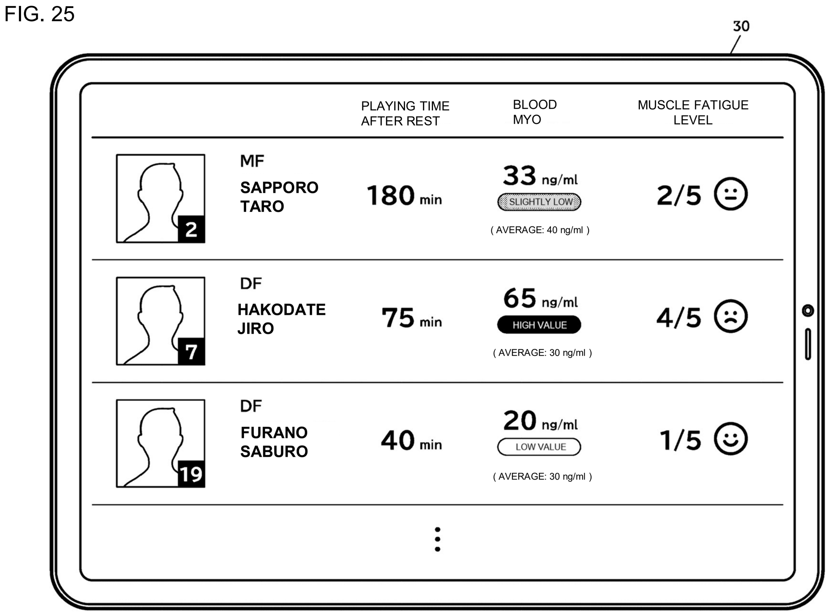

<Display Screen> FIG. 25 is a diagram showing an example of a screen displayed on the manager terminal 30. The measured myoglobin value of each player may be displayed as the muscle fatigue level in a step display (e.g., 5 levels), and may be indicated by the aspect of a corresponding object (e.g., the expression of a character). In this example, when the measured myoglobin value is a low value (1/5), a character with an expression suggesting that the muscle fatigue is small is displayed, and when the measured myoglobin value is a high value (4/5), a character with an expression suggesting that the muscle fatigue is large is displayed.

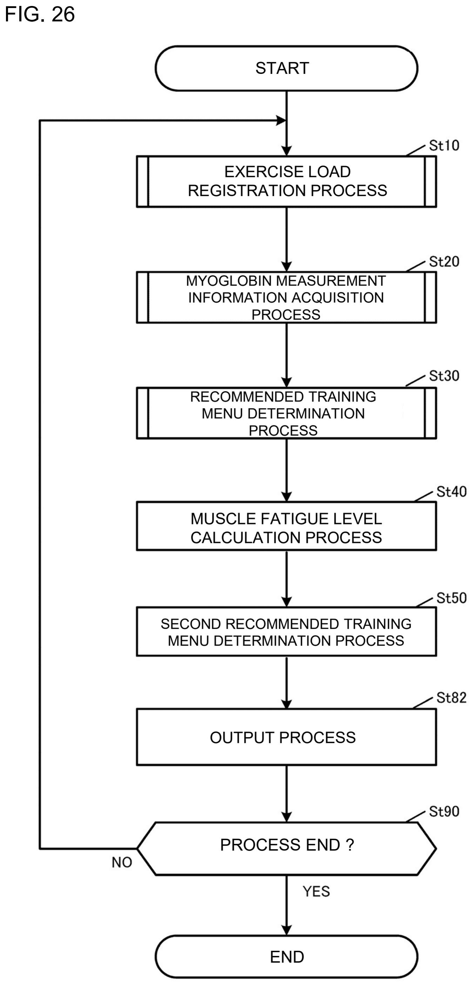

<Process> FIG. 26 is a flowchart showing an example of the flow of processes executed by each device in this embodiment, and shows the process corresponding to FIG. 17. After the recommended training menu determination process in St30, for example, the controller 11 of the server 10 performs a muscle fatigue level calculation process for calculating the muscle fatigue level for each player (St40).

Next, for example, the controller 11 of the server 10 performs a second recommended training menu determination process (St50). Specifically, the recommended training menu for each player finally proposed is determined based on, for example, the recommended training menu for each player determined in the recommended training menu determination process in St30 and the muscle fatigue level for each player calculated in St40.

Next, the controller 11 of the server 10, the controller 21 of the player terminal 20, the controller 31 of the manager terminal 30, and the like perform an output process for outputting information such as the exercise load for each player, information on the measurement of myoglobin for each player, information on muscle fatigue for each player, and the recommended training menu for each player (St82).

The information on muscle fatigue for each player may include, for example, the muscle fatigue level for each player calculated and stored in St40. Furthermore, when there is a player whose muscle fatigue level is higher than a predetermined value, alert information that warns that the muscle fatigue level is a high value for the player may be included in the information on muscle fatigue for each player, and an alert may be output by the server 10, the player terminal 20, the manager terminal 30, and the like.

Note that, for example, the muscle fatigue level calculation process (St40) may be performed after St20, the recommended training menu determination process (St30) may be performed based on the calculated muscle fatigue level, the recommended training may be determined, and the output process (St82) may be performed.

Furthermore, the server 10 may acquire the muscle fatigue level of the player not by calculating it, but by receiving the muscle fatigue level of the player from an external device that is a device different from the server 10 and calculates the muscle fatigue level of the player. Hereinafter, the acquisition of information may be the same.

<Effect of Second Embodiment> This embodiment shows a configuration in which a server (e.g., server 10) acquires the muscle fatigue level (e.g., calculates, receives) based on the exercise load of the player and the measured value of myoglobin of the player, determines the recommended training menu based on the acquired muscle fatigue level, and outputs the determined training menu to a terminal (e.g., player terminal 20, manager terminal 30). This allows the recommended training menu to be appropriately determined based on the muscle fatigue level acquired based on the exercise load of the player and the measured value of myoglobin of the player. In addition, by outputting the determined training menu to the terminal, the recommended training menu can be informed to the player or the manager.

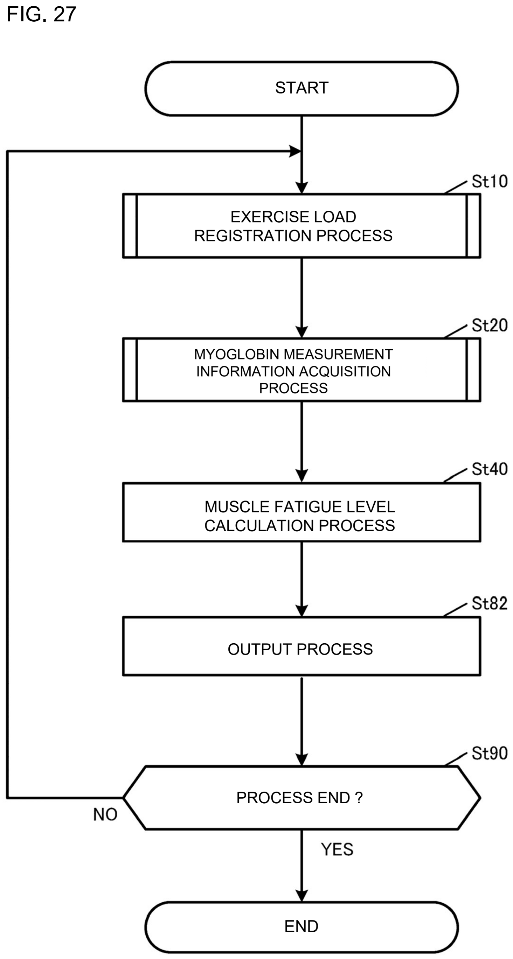

<Modification (1) of Second Embodiment> In the above process, as shown in FIG. 27, for example, the recommended training menu determination process (St30) and the second recommended training menu determination process (St50) in FIG. 26 may not be performed, and an output process (St82) for outputting information such as the exercise load for each player, information on the measurement of myoglobin for each player, and information on muscle fatigue for each player may be performed. That is, the process of determining the recommended training menu may not be performed.

Furthermore, as an example of the measurement timing for measuring blood myoglobin to calculate the muscle fatigue level in this case, it may be the measurement timing corresponding to No. "+6" in FIG. 21 (the day before the game). In this case, since the muscle fatigue level of the player can be calculated on the day before the game, it becomes possible to select the starting member for the next day's game based on the output information on muscle fatigue.

Note that this is one example, and blood myoglobin may be measured at other measurement timings, and the muscle fatigue level calculated using the result may be output.

This modification shows a configuration in which a server (e.g., server 10) acquires the muscle fatigue level based on the exercise load of the player and the measured value of myoglobin of the player, and outputs the acquired muscle fatigue level to a terminal. This allows the player or the manager to be informed by outputting the muscle fatigue level acquired based on the exercise load of the player and the measured value of myoglobin of the player to the terminal.

<Modification (2) of Second Embodiment> In the above-described embodiment, an index value (e.g., muscle recovery level) indicating the degree of recovery from muscle fatigue for each player may be calculated.

The measurement timing of myoglobin in this case may be, for example, the timing of No. "+3" in FIG. 21 (myoglobin measurement presence/absence is "present"), and the recommended training menu for the player may be determined based on the muscle recovery level of the player calculated based on the measurement result. Specifically, for example, a training menu with higher intensity may be determined as the recommended training menu for the player as the muscle recovery level of the player is higher. Furthermore, the measurement timing of blood myoglobin may be, for example, the measurement timing corresponding to No. "+6" in FIG. 21 (the day before the game), and in this case, since the muscle recovery level of the player can be calculated on the day before the game, it becomes possible to select the starting member for the next day's game with reference to this.

Furthermore, the measurement of myoglobin may be performed at the timing of No. "+1" in FIG. 21 (myoglobin measurement presence/absence is "present"), for example. Based on the muscle recovery level of the player calculated based on the measurement result, a director or a coach who supervises the player can grasp the muscle recovery level as the characteristic of each player. With reference to this, it becomes possible to plan the starting member for a plurality of games. For example, if the player had a poor muscle recovery level when the exercise load was high, but had a fast muscle recovery level when the exercise load was medium, a plan can be made such as playing for a short time (e.g., half-time only).

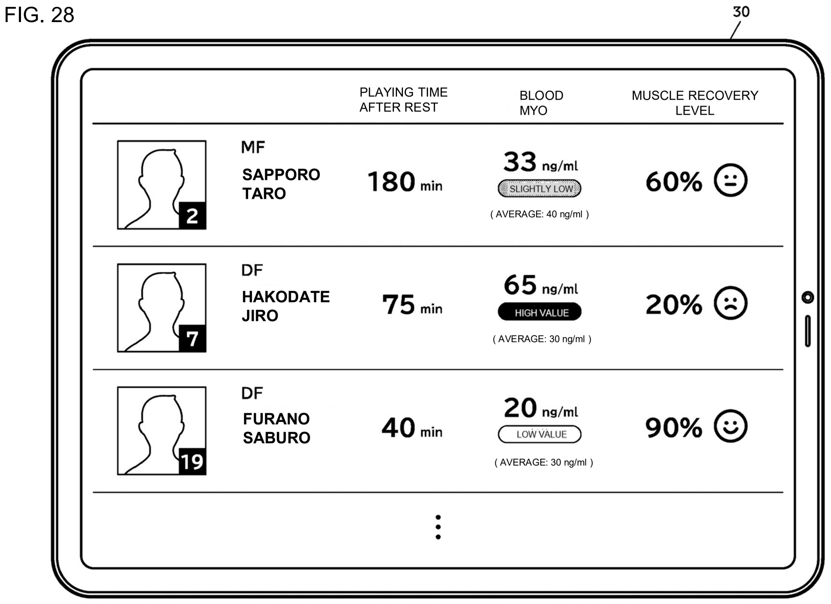

FIG. 28 is a diagram showing an example of a screen displayed on the manager terminal 30. In this screen, the muscle recovery level of each player is displayed. For example, the muscle recovery level is an index that represents to what extent the measured myoglobin value has decreased from 100%, assuming that the measured myoglobin value immediately after an event is 100%, to close to a reference value (e.g., a value before an event, the lowest past value, an average value, etc., which is a state of low muscle fatigue). A high muscle recovery level indicates that muscle fatigue has decreased, and a low muscle recovery level indicates that muscle fatigue has not decreased. If the muscle recovery level is 100%, it means that the measured myoglobin value has decreased to the reference value. In this example, since the muscle recovery level of the player with number 7 is 20%, it takes time for the measured myoglobin value to decrease to the reference value, so it can be grasped that the player is in a state where a high-intensity training menu cannot be recommended. Note that the aspect of the corresponding object (display of the character) also suggests that muscle fatigue remains. Since the muscle recovery level of the player with number 19 is 90%, the measured myoglobin value has decreased to near the reference value, and it can be grasped that the player is in a state where a high-intensity training menu can be recommended. Note that the aspect of the corresponding object (display of the character) also suggests that muscle fatigue has been resolved.

This modification shows a configuration in which a server (e.g., server 10) acquires the muscle recovery level (e.g., calculates, receives) based on the exercise load of the player and the measured value of myoglobin of the player, and outputs the acquired muscle recovery level to a terminal. This allows the player or the manager to be informed by outputting the muscle recovery level acquired based on the exercise load of the player and the measured value of myoglobin of the player to the terminal.

<Third Embodiment> A third embodiment is an embodiment related to outputting information on the general fatigue of a player and determining the recommended training menu for the player based on this information. The content of the third embodiment can be similarly applied to other embodiments and modifications.

In the previously described embodiments and modifications, stats information such as playing time, running distance, maximum running speed, sprint, and number of turns, which are exercise loads that mainly affect muscle fatigue of the player, were illustrated as the exercise load of the player. However, the exercise load is not limited to this, and for example, stats information that is a contact play element, such as the number of fouls and the number of fouls received may be used as the exercise load of the player. The contact play element may be regarded as an element related to fatigue (damage potential) to the skin, ligament, joint, bone, and the like, and may be regarded as a non-muscle fatigue element against a muscle fatigue factor.

In this embodiment, for example, the controller 11 of the server 10 calculates an index value (e.g., fatigue level, fatigue level score) indicating the degree of general fatigue of the player based on the exercise load considered to affect the fatigue of the player, and determines the recommended training menu for the player based on the calculated index value. As one approach, a comprehensive element including the muscle fatigue factor and the non-muscle fatigue factor may be regarded as a "fatigue factor", and the degree of general fatigue of the player may be quantified based on the fatigue factor.

Note that muscle fatigue factors such as playing time, running distance, maximum running speed, sprint, and number of turns can also be regarded as elements related to the general fatigue of the player, so these may also be regarded as one of the fatigue factors. Furthermore, since the contact play element may also influence muscle fatigue, the contact play element may also be regarded as one of the muscle fatigue factors.

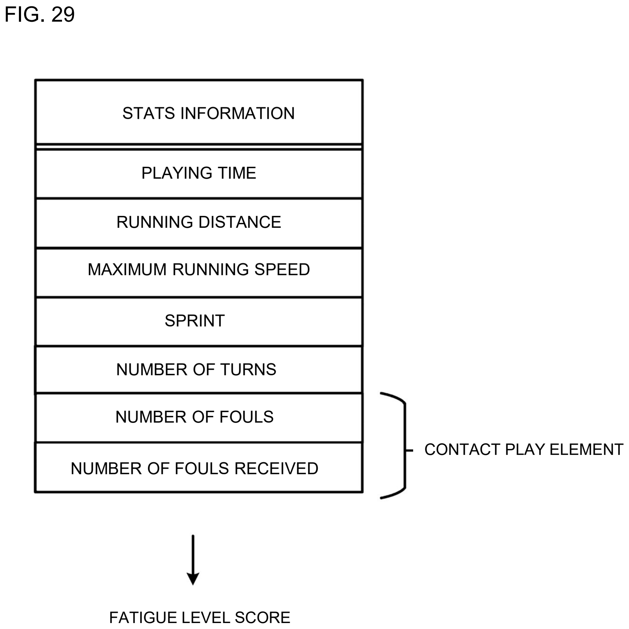

FIG. 29 is a diagram showing an example of a method for calculating a fatigue level score. For example, the controller 11 of the server 10 calculates a fatigue level score for each player based on the elements of the stats information of the player (playing time, running distance, maximum running speed, sprint, number of turns, contact play element, etc.). Specifically, for example, the fatigue level score is calculated by normalizing and adding each element. Then, for example, the controller 11 of the server 10 determines the recommended training menu for the player based on the calculated fatigue level score.

In this case, for example, the fatigue level score may be used instead of the exercise load such as the playing time previously described, and for example, the controller 11 of the server 10 may determine the recommended training menu for the player by performing threshold determination on the calculated fatigue level score of the player in the recommended training menu determination process. In this case, the recommended training menu may be determined by threshold determination using thresholds set in stages, and for example, a training menu with lower intensity (load) may be determined as the recommended training menu for the player as the fatigue level score of the player is higher.

Furthermore, weighting may be performed on each element, and the fatigue level score of the player may be calculated according to this weighting. Specifically, for example, sprint has a particularly high exercise load for a player. Therefore, for example, the weight for sprint may be set higher than other elements to calculate the fatigue level score.

Furthermore, for example, the controller 11 of the server 10 may determine the recommended training menu for the player based on the myoglobin score of the player and the fatigue level score of the player.

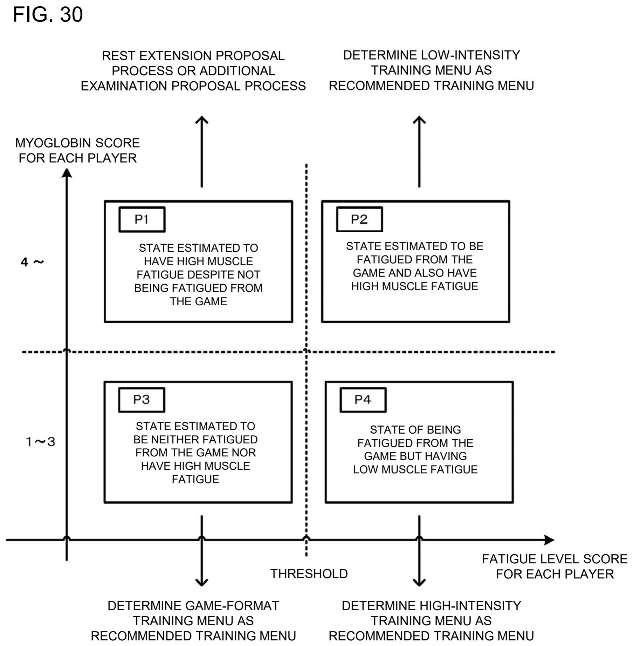

FIG. 30 is a diagram for explaining an example of a method for determining the recommended training menu in this case. This diagram shows a two-axis format table in which the vertical axis is the myoglobin score for each player and the horizontal axis is the fatigue level score for each player. The myoglobin score for each player on the vertical axis is classified into two (score 4 or more, score 1 to 3), and the fatigue level score for each player on the horizontal axis is classified into two by a threshold.

Pattern P1 is a pattern where the myoglobin score is 4 or more and the fatigue level score is less than the threshold. This pattern is a pattern corresponding to a "state where the player is estimated to not be fatigued from the game but the muscle fatigue is large". In this pattern, for example, the controller 11 of the server 10 may perform a process of proposing an extension of rest to the player (rest extension proposal process) or a process of proposing an additional examination to the player (additional examination proposal process). In this case, the controller 11 of the server 10 may transmit proposal information to, for example, the player terminal 20 or the manager terminal 30 of the player so that the proposal information is displayed on these terminals.

Pattern P2 is a pattern where the myoglobin score is 4 or more and the fatigue level score is equal to or greater than the threshold. This pattern is a pattern corresponding to a "state where the player is fatigued from the game and the muscle fatigue is also estimated to be large". In this pattern, for example, the controller 11 of the server 10 may determine a low-intensity training menu (the training menu with the lowest intensity) as the recommended training menu for the player in the recommended training menu determination process.

Pattern P3 is a pattern where the myoglobin score for each player is 1 to 3 and the fatigue level score is less than the threshold. This pattern is a pattern corresponding to a "state where the player is estimated to not be fatigued from the game and the muscle fatigue is also small". In this pattern, for example, the controller 11 of the server 10 may determine a game-format training menu (the training menu with the highest intensity) as the recommended training menu for the player in the recommended training menu determination process.

Pattern P4 is a pattern where the myoglobin score for each player is 1 to 3 and the fatigue level score is equal to or greater than the threshold. This pattern is a pattern corresponding to a "state where the player is fatigued from the game but the muscle fatigue is estimated to be small". In this pattern, for example, the controller 11 of the server 10 may determine a high-intensity training menu (the training menu with the second highest intensity) as the recommended training menu for the player in the recommended training menu determination process.

Note that, in FIG. 30, for example, the horizontal axis may be the exercise load for each player, and the recommended training menu for the player may be determined in the same manner as above based on the exercise load of the player and the myoglobin score.

<Effect of Third Embodiment> This embodiment shows a configuration in which a server (e.g., server 10) acquires information on the fatigue of the player (e.g., fatigue level score) based on the exercise load of the player and the measured value of myoglobin of the player (e.g., calculates, receives), determines the recommended training menu based on the acquired information, and outputs the determined training menu to a terminal (e.g., player terminal 20, manager terminal 30). This allows the recommended training menu to be appropriately determined based on the information on the fatigue of the player acquired based on the exercise load of the player and the measured value of myoglobin of the player. In addition, by outputting the determined training menu to the terminal, the recommended training menu can be informed to the player or the manager.

<Modification (1) of Third Embodiment> In the above-described embodiment, similar to Modification (1) of the second embodiment, the recommended training menu determination process may not be performed, and an output process for outputting various information including the fatigue level score may be performed.

<Modification (2) of Third Embodiment> In the above-described embodiment, for example, an index value (e.g., fatigue recovery level) indicating the degree of fatigue or the degree of recovery from fatigue of the player may be calculated based on the heart rate information of the player, and this may be output to determine the recommended training menu. Alternatively, the recommended training menu determination process may not be performed, and an output process for outputting various information including the fatigue level score may be performed. Note that, as described later, the heart rate of the player may be acquirable by, for example, a heart rate measurement function of a wearable device worn by the player.

In this case, for example, the fatigue recovery level of the player may be calculated and estimated based on the information regarding the recovery of the player. For example, the number of times the heart rate of the player decreased from the 90% range to the 70% range during a game (number of recoveries) and the average time required for it (average recovery time) are calculated. Then, the fatigue recovery level of the player may be calculated and estimated based on how long the recovery time on the measurement date is compared to the average recovery time of the player. A player who can continuously perform high-intensity plays will have a higher number of recoveries and a shorter average recovery time (see, e.g., "https://know-s.com/2022/04/08/907/"). Furthermore, for example, the fatigue recovery level of the player may be calculated in the same manner as above based on the time during which the heart rate is in a state higher than a predetermined threshold.

Furthermore, for example, the fatigue recovery level of the player may be calculated based on the heart rate recovery of the player calculated according to the following formula: Heart rate recovery = Average heart rate of the player at the time of the game - Average heart rate of the player at the time of measurement.

Based on the fatigue recovery level of the player calculated in this way, the controller 11 of the server 10 may determine the recommended training menu for the player, similar to the previously described embodiments, for example. Specifically, a training menu with higher intensity may be determined as the recommended training menu for the player as the fatigue recovery level of the player is greater. Alternatively, an output process for outputting the fatigue recovery level of the player may be performed.

<Fourth Embodiment> A fourth embodiment is an embodiment related to a method for acquiring the exercise load of the player previously described (e.g., elements of stats information). The content of the fourth embodiment can be similarly applied to other embodiments and modifications.

In this embodiment, for example, in the exercise load registration process (St10) of the various flowcharts previously described, the controller 11 of the server 10 may directly acquire the exercise load of the player measured by a wearable device (orthosis) worn by the player from the wearable device, or indirectly acquire it via the player terminal 20 or the manager terminal 30, and store it in the exercise load data.

As the wearable device, for example, devices such as a vest-type device, a pouch-type device, a bra-type device, a watch-type device, and a shoe-type device can be used. Note that when measuring the exercise load of the player during a game, a device permitted to be worn during the game may be used.

Furthermore, the wearable device may be a device configured to be capable of measuring and/or calculating information such as position, running distance, speed, acceleration/deceleration, body tilt, change of direction, and heart rate as information regarding the exercise load of the player, by including units (sensors) such as, for example, a GNSS unit (Global Navigation Satellite System unit), an IMU (Inertial Measurement Unit), a direction sensor (e.g., geomagnetic sensor), and a heart rate sensor (e.g., blood flow sensor).

For example, the controller 11 of the server 10 may calculate the exercise load such as the maximum running speed, sprint, and number of turns previously described based on the information acquired from the above-described wearable device. Furthermore, for example, the controller 11 of the server 10 may calculate the fatigue recovery level previously described (recovery level based on heart rate) based on the heart rate information acquired from the wearable device.

<Others> Embodiments to which the present invention can be applied are not limited to the above-described embodiments. For example, various sports other than soccer may be applied as the sport, and a system for supporting health management of players of various sports may be configured.

Furthermore, at least a part of the processing to be performed by the server 10 in the above-described embodiments may be performed by a terminal. Conversely, at least a part of the processing to be performed by a terminal in the above-described embodiments may be performed by the server 10.

Furthermore, the processes described in the above-described embodiments may be implemented by one server, or may be shared and implemented by a server system constituted by a plurality of servers. Note that a system constituted by one or more servers may be defined as a server system, and the server may be the server system.

Furthermore, the present invention is not limited to a client-server system, and may be implemented by a system such as a distributed system that provides the functions of the server or server system to the terminal.

### Remarks

The present disclosure includes following items.

A system for supporting health management of an athletic player, comprising:
a measuring device configured to measure myoglobin contained in blood of the player; and
a server configured to record an exercise load of the player and a measured value of the myoglobin of the player,
wherein the server is configured to provide the exercise load of the player and the measured value of the player in response to a request from a terminal.

The system according to item 1, wherein the server is configured to generate a reference value of myoglobin for each player based on a plurality of measured values, and evaluate a newly measured myoglobin value based on the reference value.

The system according to item 2, wherein the server is configured to calculate the reference value based on the measured value measured after a predetermined time has elapsed from a game.

The system according to item 2, wherein the server is configured to transmit an evaluation result based on the measured value and the reference value to the terminal associated with an account of the player.

The system according to item 1, wherein the measuring device is configured to measure the myoglobin by:
mixing a reagent including a labeled antibody with the blood to label the myoglobin with the labeled antibody; and
optically detecting the labeled myoglobin.

The system according to item 5, wherein the measuring device is configured to separate the blood into a plasma and a blood cell, and to mix the plasma with the reagent.

The system according to item 6, wherein the measuring device is configured to output the measured value after a predetermined time has elapsed from the measurement start.

The system according to item 1, wherein the system is further configured to output an alert for the player when the measured value is higher than a predetermined value.

The system according to item 1, wherein the server is configured to determine a recommended training menu based on the exercise load and the measured value, and to output the training menu to the terminal.

The system according to item 1, wherein the server is configured to acquire a muscle fatigue level based on the exercise load and the measured value, to determine a recommended training menu based on the muscle fatigue level, and to output the training menu to the terminal.

The system according to item 1, wherein the server is configured to acquire a muscle fatigue level based on the exercise load and the measured value, and to output the muscle fatigue level to the terminal.

The system according to item 1, wherein the server is configured to acquire a muscle recovery level based on the exercise load and the measured value, and to output the muscle recovery level to the terminal.

A method for supporting health management of an athletic player, comprising:
measuring myoglobin contained in blood of the player;
recording an exercise load of the player and a measured value of the myoglobin of the player; and
providing the exercise load of the player and the measured value of the player in response to a request from a terminal.

A computer-based health monitoring system for an athletic player, comprising:
a measuring device configured to measure myoglobin contained in blood of the player, wherein the measuring device comprises:
   (i) a separator configured to separate plasma from the blood;
   (ii) a reaction part configured to react the plasma with a reagent to prepare a measurement sample in which the myoglobin is labeled by the reagent;
   (iii) a detection part configured to detect optical signal from the myoglobin contained in the measurement sample;
   (iv) a first processor configured to obtain a measurement value indicative of concentration of the myoglobin in the blood, the first processor is configured to convert the signal into the concentration by using a calibration curve; and
a server configured to record an exercise load of the player and a measured value of the myoglobin of the player and to provide the exercise load of the player and the measured value of the player in response to a request from a terminal,
wherein the server comprises a second processor programmed to store a combination of the exercise load and the measurement value associated with an identification information of the athletic player in a storage,
wherein the second processor is configured to retrieve the exercise load and the measured value from the storage with reference to the identification information of the athletic player and provide the retrieved data to the terminal.

## Claims

1. A system (1) for supporting health management of an athletic player, comprising:
a measuring device (40) configured to measure myoglobin contained in blood of the player; and
a server (10) configured to record an exercise load of the player and a measured value of the myoglobin of the player,
wherein the server is configured to provide the exercise load of the player and the measured value of the player in response to a request from a terminal (20, 30).

2. The system according to claim 1, wherein the server is configured to generate a reference value of myoglobin for each player based on a plurality of measured values, and evaluate a newly measured myoglobin value based on the reference value.

3. The system according to claim 2, wherein the server is configured to calculate the reference value based on the measured value measured after a predetermined time has elapsed from a game.

4. The system according to claim 2 or 3, wherein the server is configured to transmit an evaluation result based on the measured value and the reference value to the terminal associated with an account of the player.

5. The system according to any one of claims 1-4, wherein the measuring device is configured to measure the myoglobin by:
mixing a reagent including a labeled antibody with the blood to label the myoglobin with the labeled antibody; and
optically detecting the labeled myoglobin.

6. The system according to claim 5, wherein the measuring device is configured to separate the blood into a plasma and a blood cell, and to mix the plasma with the reagent.

7. The system according to claim 6, wherein the measuring device is configured to output the measured value after a predetermined time has elapsed from the measurement start.

8. The system according to any one of claims 1-7, wherein the system is further configured to output an alert for the player when the measured value is higher than a predetermined value.

9. The system according to any one of claims 1-8, wherein the server is configured to determine a recommended training menu based on the exercise load and the measured value, and to output the training menu to the terminal.

10. The system according to any one of claims 1-9, wherein the server is configured to acquire a muscle fatigue level based on the exercise load and the measured value, to determine a recommended training menu based on the muscle fatigue level, and to output the training menu to the terminal.

11. The system according to any one of claims 1-10, wherein the server is configured to acquire a muscle fatigue level based on the exercise load and the measured value, and to output the muscle fatigue level to the terminal.

12. The system according to any one of claims 1-11, wherein the server is configured to acquire a muscle recovery level based on the exercise load and the measured value, and to output the muscle recovery level to the terminal.

13. A method for supporting health management of an athletic player, comprising:
measuring myoglobin contained in blood of the player;
recording an exercise load of the player and a measured value of the myoglobin of the player; and
providing the exercise load of the player and the measured value of the player in response to a request from a terminal.

14. A computer-based health monitoring system for an athletic player, comprising:
a measuring device configured to measure myoglobin contained in blood of the player, wherein the measuring device comprises:
(i) a separator configured to separate plasma from the blood;
(ii) a reaction part configured to react the plasma with a reagent to prepare a measurement sample in which the myoglobin is labeled by the reagent;
(iii) a detection part configured to detect optical signal from the myoglobin contained in the measurement sample;
(iv) a first processor configured to obtain a measurement value indicative of concentration of the myoglobin in the blood, the first processor is configured to convert the signal into the concentration by using a calibration curve; and
a server configured to record an exercise load of the player and a measured value of the myoglobin of the player and to provide the exercise load of the player and the measured value of the player in response to a request from a terminal,
wherein the server comprises a second processor programmed to store a combination of the exercise load and the measurement value associated with an identification information of the athletic player in a storage,
wherein the second processor is configured to retrieve the exercise load and the measured value from the storage with reference to the identification information of the athletic player and provide the retrieved data to the terminal.
